(19) Europäisches Patentamt
European Patent Office
Office européen des brevets

(11) EP 2 311 465 A1

(12) # EUROPEAN PATENT APPLICATION

(43) Date of publication:
**20.04.2011 Bulletin 2011/16**

(21) Application number: **10178007.0**

(22) Date of filing: **16.04.2004**

(51) Int Cl.:
***A61K 31/56*** (2006.01) ***A61K 31/565*** (2006.01)
***A61K 31/568*** (2006.01) ***A61K 45/00*** (2006.01)
***A61K 9/06*** (2006.01) ***A61K 9/08*** (2006.01)
***A61K 47/40*** (2006.01) ***A61P 5/00*** (2006.01)
***A61P 27/02*** (2006.01) ***A61P 27/06*** (2006.01)
***A61P 27/10*** (2006.01) ***C07J 1/00*** (2006.01)

(84) Designated Contracting States:
**AT BE BG CH CY CZ DE DK EE ES FI FR GB GR HU IE IT LI LU MC NL PL PT RO SE SI SK TR**

(30) Priority:
**18.04.2003 JP 2003114969**
**12.05.2003 JP 2003132910**
**19.09.2003 JP 2003329165**
**14.10.2003 JP 2003354135**
**31.03.2004 PCT/JP2004/004709**

(62) Document number(s) of the earlier application(s) in accordance with Art. 76 EPC:
**04727990.6 / 1 621 199**

(71) Applicant: **Advanced Medicine Research Institute**
**Setagaya-ku**
**Tokyo 158-0085 (JP)**

(72) Inventor: **Okamoto, Shinseiro**
**Setagaya-ku Tokyo 158-0085 (JP)**

(74) Representative: **Hiebl, Inge Elisabeth**
**Kraus & Weisert**
**Patent- und Rechtsanwälte**
**Thomas-Wimmer-Ring 15**
**80539 München (DE)**

Remarks:
This application was filed on 21-09-2010 as a divisional application to the application mentioned under INID code 62.

(54) **Remedies for diseases to be applied to eye**

(57) An agent for treating cataract which contains sexual steroid hormone such as estrogen or its metabolites, its derivative, structural analogues thereof, estrogen acting substance or SERM or non-feminizing estrogen (non-hormonal estrogen) and has a form of eye drops or eye washes, etc. An agent for treating cataract which has excellent treatment effects, reduced in side action can be provided.

EP 2 311 465 A1

## Description

Technical field

The present invention relates to an agent for treating eye diseases containing sexual steroid hormones (for example, estrogen), a selective estrogen receptor modulator, non-feminizing estrogen (non-hormonal estrogen) or other substances having an estrogen action, particularly to an agent for treating eye diseases in a form of eye drops/eye washes, etc.

Background art

According to a hormone replacement therapy (HRT) taking estrogen alone, or a combination of estrogen and progesterone orally, it has been reported a possibility of improving symptoms of various kinds of eye diseases such as age related macular degeneration (AMD), macular hole, glaucoma, diabetic retinopathy, senile cataract, etc.
However, HRT sometimes causes potent side effects such as breast cancer, uterine cancer, gynecomastia, impotence, etc. since estrogen (or estrogen and progesterone) is administered to whole body. Accordingly, HRT cannot easily be used for treatment of various kinds of diseases, particularly for treatment of eye diseases in spite of possessing a possibility of remedy.
Occurrence of glaucoma which is an eye disease has been said to be 1 person per 30 persons among the people over the age of 40. However, according to the research report of Japan Glaucoma Association in 2002, it occurs 1 person per 17 persons among the people over the age of 40, whereby a number of patients of glaucoma is increasing. Reaching an aging society, increase in a number of patients of eye diseases including glaucoma can be expected in the future.
Eye drops for treatment of eye diseases such as glaucoma conventionally been used lower intraocular pressure, but are insufficient in remedy for narrowing of visual field, and also, there are some cases to cause hyperemia of eyes or corneal erosion. Also, it is required to be dropped to eyes several times per day so that it is troublesome.
An object of the present invention is to provide an agent for treatment of diseases by eye drops, etc., which overcome the problem involved in HRT such as side effect, etc.
Also, another object of the present invention is to provide a basic agent for treatment of eye diseases such as glaucoma, cataract, etc., which has a prolonged time of treatment effects such as improvement in visual field, lowering in intraocular pressure, etc., and can give effects with a dropping time of once per day or once per 2 or 3 days.

Disclosure of the invention

The present invention relates to an agent for treating eye diseases comprising sexual steroid hormones (for example, estrogen), a selective estrogen receptor modulator, non-feminizing estrogen (non-hormonal estrogen) or other substances having an estrogen action.
The sexual steroid hormone(s) to be contained in an agent for treating diseases of the present invention is/are hormone(s) mainly synthesized or secreted in gonad such as ovary, testis, etc., or in placenta, and in the present invention, a synthetic substance having its action, a derivative thereof and metabolites thereof are also contained. The sexual steroid hormone is roughly classified into androgen, estrogen and gestagen. It is preferably a sexual steroid hormone having a carbon number of 18 to 21.
As the androgen, there may be exemplified by, for example, testosterone, androstenedione, dehydroepiandrosterone, androsterone, androstanedione, methyl testosterone and derivatives thereof or structural analogues thereof, and testosterone and derivatives thereof or structural analogues thereof are preferred.
As the estrogen, there may be exemplified by 17β-estradiol, estrone, estriol, equilin, equilenin, ethynyl estradiol etc., and its derivatives, metabolites, etc., and 17β-estradiol and derivatives thereof, and metabolites thereof are preferred.
As the other substances having an estrogen action, there may be exemplified by phytoestrogen, etc., and its derivatives and metabolites thereof.
As the non-feminizing estrogen, there may be mentioned, for example, a compound of the following formula:

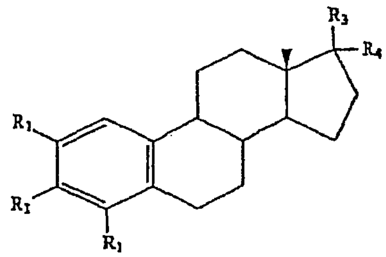

(wherein $R_1$ represents H, OH, $OR_2$ or $R_2$, $R_2$ represents a $C_1$ to $C_6$ alkyl, $R_3$ represents OH, =O or $R_1$, $R_4$ represents $R_1$, C≡CH or $C=CH_2$) described in USP No. 5,521,168, particularly 2-methoxyestradiol, 2-hydroxyestradiol, 4-methoxyestradiol, 2-methoxyestradiol 3-methyl ether, 2-methoxyestrone and 2-hydroxyestrone, etc. Also, there may be mentioned a compound of the following formula:

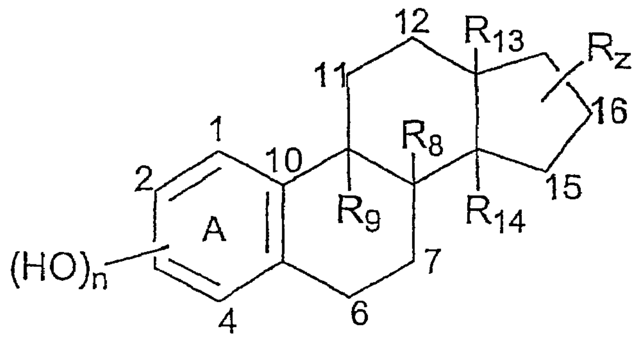

(wherein the compound can have one or more double bond(s) which conjugate(s) aromatic ring A between carbons 6 and 7, 8 and 9, or 9 and 11, and in such a case, either one of $R_8$ and $R_9$ or both of them do not exist, n is 1 to 4, when $R_8$ and $R_9$ exist, they are hydrogen or alkyl, $R_{13}$ represents hydrogen, substituted or unsubstituted hydrocarbyl, halogen, amide, sulfate or nitrate, $R_{14}$ represents hydrogen or alkyl, $R_z$ represents a substituted or unsubstituted cycloalkyl, cycloalkenyl, heterocycloalkyl or heterocycloalenyl) described in WO 02/40032 A2, particularly ZYC-1, 2, 4, 9, 10, 11, 12, 13, 27 and 28, etc. Also, there may be mentioned ZYC-5 described in Neurobiology of Disease 9, 282-293 (2002).

As the gestagen, progesterone and derivatives thereof or structural analogues thereof are preferred.

The sexual steroid hormone is preferably one or more kinds selected from the group consisting of estrogen, testosterone, progesterone and androgen, and derivatives thereof, metabolites and structural analogues thereof.

Particularly preferred as the sexual steroid hormones are 17β-estradiol and derivatives thereof, metabolites thereof, and structural analogues thereof.

The sexual steroid hormones such as 17β-estradiol, etc., become suspension when they are mixed with water. Accordingly, in an agent for treating diseases of the present invention, it is preferred to make sexual steroid hormones such as 17β-estradiol water-soluble, or, make water-solubilize sexual steroid hormones with a surfactant, particularly in the points of effects, it is preferred to make water-solubilize 17β-estradiol or derivative thereof, or metabolites thereof.

17β-Estradiol is hydrophobic and has low solubility in water, so that even when it is mixed with distilled water, it makes aggregates with ununiform size so that a particle size becomes large, preservation stability is poor, whereby setting of a concentration to be desired is difficult. Accordingly, it is necessary to carry out a certain device to prepare stable eye drops from a difficultly soluble substance in water such as 17β-estradiol, etc., which can set a desired concentration. It is necessary to provide a safe and useful method which can ensure stability of a medical agent, and not damage physiological and chemical characteristics of cornea and conjunctiva. Also, it is necessary to not causing irritation or unpleasant feeling at the time of use.

Heretofore, for preparing eye drops from a difficultly soluble substance, there have been attempted to use a method of adding an additive, oil, alcohol, etc., or increasing a viscosity of eye drops, but they involved defects that unpleased feeling occurs at the time of drop, and the like.

To the contrary, in the present invention, it is preferred to employ a method in which a medical agent is included by using cyclodextrin to make soluble in water. Cyclodextrin is oligosaccharide of glucose, outside thereof is hydrophilic

and inside thereof (cavity) is hydrophobic, and by making an inclusion compound with a hydrophobic medical agent such as 17β-estradiol, the agent can be water-solubilized and stabilized, and eye drops with no unpleasant feeling at the time of use can be obtained so that it is suitable for preparing eye drops. In the cyclodextrin, there have been generally known to exist three kinds of α, β and γ.

Among the cyclodextrins, β-cyclodextrin involves problems of having hemolytic property, renal toxic property due to low water-solubility, etc., so that it is wise to use derivatives of β-cyclodextrin as mentioned below.

In the β-cyclodextrin derivatives, there are hydrophilic cyclodextrin and hydrophobic cyclodextrin, and hydrophilic ones are suitable for eye drops. In the hydrophilic cyclodextrin, there are methylated cyclodextrin and hydroxyalkylated cyclodextrin, and it can be found from the results of experiments in animals and human being as mentioned below that methylated cyclodextrin is not suitable due to irritation of eyes, damage to cornea or conjunctiva, or a problem of hemolysis. On the other hand, hydroxyalkylated cyclodextrin has high safety, no irritation to eyes, no damage to corneal epithelium and excellent for preparing eye drops.

In the hydroxy alkylated cyclodextrin, hydroxyethylcyclodextrin, hydroxypropylcyclodextrin, etc. are included, in particular, 2-hydroxypropyl-β-cyclodextrin is most preferred in the points that it is excellent in water-solubility of a inclusion compound or stability of a medical agent, and less irritation or living body toxicity.

γ-Cyclodextrin and its derivative can be preferably used. α-Cyclodextrin has not so high water-solubility of 17β-estradiol by inclusion, so that γ-cyclodextrin is preferred.

Hydroxypropyl-y-cyclodextrin, sulfonyl-β-cyclodextrin, sulfobutyl ether-β-cyclodextrin, or δ-cyclodextrin can be used.

Water-solubilization of sexual steroid hormones such as 17β-estradiol, etc. can be carried out, for example, according to the conventional manner, by using sexual steroid hormones as a guest compound, and included in a host compound such as a cyclic compound including α-, β- or γ-cyclodextrin, etc. or their derivative, etc. The water-solubilized sexual steroid hormones are good in solubility in water, and can transfer and impregnate an effective ingredient into a tissue of eyes (particularly retina) by dropping, so that remedy effects are high, a concentration in blood in whole body can be depressed which means less side effects, and also, feeling at the time of use is good and no unpleasant feeling, whereby it is particularly suitable for eye drops.

It is confirmed that water-solubilized 17β-estradiol is transferred to an objective portion in eyes by dropping, and activated cells (see Examples 1 to 6 mentioned below).

In the selective estrogen preceptor modulator (SERM) to be contained in an example for treatment of diseases of the present invention, there may be mentioned, for example, raloxifene, arzoxifene, etc.

Structural analogues of estrogen, a selective estrogen receptor modulator, a non-feminizing estrogen (non-hormonal estrogen) or other substance(s) having estrogen action can be considered to be effective for treatment of diseases at the objective portion for treatment by heightening transferability into an objective tissue in eyes by making it water-solubilizing as the water-solulubilized 17β-estradiol.

Diseases capable of treating in the present invention are not specifically limited, and for example, there may be mentioned glaucoma, high tension glaucoma, normal tension glaucoma, neovascular senile macular degeneration, senile macular diseases, central chorioretinopathy (chorioretinitis), senile macular degeneration, macular hole, cataract, senile cataract, chorioretinal hemorrhage, central retinal artery occlusion, arteriosclerosis of retinal artery, photopsia, diabetic retinopathy, chorioretinal atrophy, retinal and choroidal neovascular diseases, cataract due to removal of ovary, cataract due to TGF p, macular epiretinal membrane (macular fibrosis), retinal tear, retinal detachment, retinitis proliferans, pigmentary retinal degeneration, keratitis, corneal opacity, corneal erosion, detachment of corneal epithelium, corneal ulcer, corneal endothelial cell degeneration and dystrophy or loss of endothelial cell, corneal dystrophy (degeneration), epidemic keratoconjunctivitis, chalazion, iritis, uveitis, autoimmune disease, chorioretinitis, iridocyclitis, asthenopia, narrowing of visual field due to various kinds of diseases, optic nerve atrophy, optic neuritis, (anterior) ischemic optic neuropathy, lowering in dynamic visual activity, abnormal color vision, refractive error such as presbyopia, myopia, hyperopia, astigmatism, etc., and central nerve diseases such as Alzheimer's diseases, Parkinson's disease, ALS, etc., and psychosis such as schizophrenia and manic-depressive psychosis, hysteria, diseases due to cerebral pituitary gland disorder and unbalance of hormons, diseases due to gene disorder and diseases due to immune disorder. In glaucoma, Posner-Schlossmans' Syndrome is included, and in corneal dystrophy (degeneration), cornea endothelial cell dystrophy (degeneration) and dropping, corneal granular degeneration (Groenouw Type 1, Groenouw Type 2), corneal band shaped keratopathy.

The agent for treatment of the present invention is a material to directly apply to eyes, and the form can be, for example, eye drops, eye washes, ointments, conjunctiva injections or contact lens adsorbents.

In the agent for treating eye diseases of the present invention, sexual steroid hormone (e.g., estrogen), a selective estrogen receptor modulator, non-feminizing estrogen (non-hormonal estrogen) or other substance(s) having an estrogen action is/are directly administered to eye tissues by dropping, so that there is no fear of causing side effect caused by increasing a concentration of sexual steroid hormone, etc., in blood of a whole body which occurs by the conventional HRT treating through circulatory system, a time of continuing an effect is long, whereby the diseases can

be effectively treated with a small number of dropping times. (1) In particular, it can markedly improve narrowing of visual field which is impossible by the conventional treatment method, so that (2) it can carry out treatment of not only high tension glaucoma, (3) but also treatment of normal tension glaucoma particularly effectively, in which there is no treatment method as of today, since there is an improved effect in visual field.

Brief description of the drawings

Fig. 1 is a photograph of eye drops A of Example 1.
Fig. 2 is a photograph of eye drops B of Example 1.
Fig. 3 is a graph showing a transferred concentration of 17β-estradiol into anterior chamber after dropping eye drops A to C with a lapse of time.
Fig. 4 is a photograph of anterior portion of mouse in Control group (Control), UV ray irradiated pre-administered group (Pre 0.01 g/l and 0.1 g/l) and UV ray irradiated post-administered group (Post 0.01 g/l and 0.1 g/l) in Example 4.
Fig. 5 is a microscopic photograph of retina of mouse to which distilled water was dropped in Example 5.
Fig. 6 is a microscopic photograph of retina of mouse to which water-soluble 17β-estradiol-containing eye drops was dropped in Example 5.
Fig. 7 is an estrogen receptor antibody positive image at retina.
Fig. 8 is an estrogen receptor antibody positive image at lens.
Fig. 9 is an estrogen receptor antibody positive image at cornea.
Fig. 10 is an estrogen receptor antibody positive image at ciliary body.
Fig. 11 is an image at retinal external granular (nuclear layer) cells when estrogen was not added and an antibody not added.
Fig. 12 is an estrogen receptor antibody positive image at retinal external granular (nuclear layer) cells when 17β-estradiol was not added and an antibody added.
Fig. 13 is an image at retinal external granular (nuclear layer) cells when 17β-estradiol was added and an antibody not added.
Fig. 14 is an estrogen receptor antibody positive image at retinal external granular (nuclear layer) cells when 17β-estradiol was added and an antibody added.
Fig. 15 shows a concentration of 17β-estradiol (0 to 100 μg/ml) dropped and a number of cells to which estrogen receptor has transferred to nucleus.
Fig. 16 shows a number of cells to which estrogen receptor has transferred to nucleus with a lapse of time after dropping of 17β-estradiol.
Fig. 17 is a drawing showing a visual field of right eye of a patient of Example 9 before treatment.
Fig. 18 is a drawing showing a visual field of left eye of a patient of Example 9 before treatment.
Fig. 19 is a drawing showing a visual field of right eye of a patient of Example 9 after treatment.
Fig. 20 is a drawing showing a visual field of left eye of a patient of Example 9 after treatment.
Fig. 21 is a drawing showing a visual field of right eye of a patient of Example 10 before treatment.
Fig. 22 is a drawing showing a visual field of left eye of a patient of Example 10 before treatment.
Fig. 23 is a drawing showing a visual field of right eye of a patient of Example 10 after treatment.
Fig. 24 is a drawing showing a visual field of left eye of a patient of Example 10 after treatment.
Fig. 25 is a drawing showing a relation between a term of dropping and visual acuity of a patient in Example 11.
Fig. 26 is a drawing showing change in near point before and after dropping of a patient in Example 12.
Fig. 27 is a drawing showing a visual field of right eye of a patient of Example 13 before treatment.
Fig. 28 is a drawing showing a visual field of left eye of a patient of Example 13 before treatment.
Fig. 29 is a drawing showing a visual field of right eye of a patient of Example 13 after treatment.
Fig. 30 is a drawing showing a visual field of left eye of a patient of Example 13 after treatment.
Fig. 31 is a drawing showing dynamic visual acuity of a patient in Example 26.

Best mode for carrying out the invention

The treatment agent of the present invention can be prepared according to the method conventionally used in the same manner as in this kind of the general medical agents. In case of eye drops or eye washes, for example, sexual steroid hormone (e.g., estrogen), a selective estrogen receptor modulator (e.g., raloxifene), non-feminizing estrogen (non-hormonal estrogen) (e.g., ZYC-5) or other substance(s) having an estrogen action which are all water-solubilized is/are dissolved in sterilized distilled water, BSS plus, physiological saline, etc., with a predetermined concentration to obtain a preparation.

In the treatment agent of the present invention, sexual steroid hormone (e.g., estrogen), a selective estrogen receptor modulator (e.g., raloxifene), non-feminizing estrogen (non-hormonal estrogen) (e.g., ZYC-5) or other substance (s) having an estrogen action is/are required to be contained in an amount effective for the treatment. An amount effective for the treatment is, in the case of a liquid agent, for example, in a concentration of 0.1 μg to 100 g/l, preferably 0.5 μg to 1 g/l, particularly preferably 1 μg to 500 mg/l, and in the case of a solid agent, there may be mentioned, for example, 0.1 μg to 100 g/kg, preferably 0.5 μg to 10 g/kg, particularly preferably 1 μg to 5 g/kg.

The treatment agent of the present invention can control a time during which the effect develops or a time during which the effect appears by changing a concentration of the sexual steroid hormone (e.g., estrogen), the selective estrogen receptor modulator, non-feminizing estrogen (non-hormonal estrogen) or other substance(s) having an estrogen action in the preparation, a number of times for administration, an administered dose, or else selecting a method of administration of the medicinal agent (for example, eye drops, eye washers, or ointment).

For the treatment of the present invention, if necessary, conventionally used additives such as excipients, carriers, pH controllers, isotonic agents, preservatives, glutathione, glucose, various kinds of salt(s), stabilizers, refrigerants, antioxidants, antiseptic agents, etc. may be added.

Also, hydroxypropylmethyl cellulose, carboxymethyl cellulose or its sodium salt, polypyrrolidone, polyvinylpyrrolidone (this is added and heated), etc., may be added.

Example

Next, the present invention is explained by referring to Examples, but the present invention is not limited by these Examples.

Example 1

As described below, eye drops were prepared from 17β-estradiol and inclusion products thereof with various kinds of cyclodextrins. Various kinds of cyclodextrins were used those available from SIGMA Co. (MO, USA), and 17β-estradiol was used those available from CALBIOCHEM AG (Germany).

Eye drops A: eye drops of 17β-estradiol

17β-estradiol was dissolved in distilled water with a concentration of 1 mg/ml. When this was allowed to stand, it caused aggregated clogs with at random size and became dispersion. This is made eye drops A (Fig. 1) without raising a viscosity or adding alcohol.

Eye drops B: eye drops of inclusion material of 2-hydroxypropyl-β-cyclodextrin and 17β-estradiol

In 13.3 mM of 2-hydroxypropyl-β-cyclodextrin was included 3.67 mM (1 mg/ml) of 17β-estradiol, and the inclusion material was dissolved in distilled water to prepare a transparent uniform aqueous solution (Fig. 2). This aqueous solution was made eye drops B (molar ratio (3.62: 1)).

Eye drops C: eye drops of inclusion material of α- + γ-cyclodextrin and 17β-estradiol

In α-cyclodextrin was included 3.67 mM (1 mg/ml) of 17β-estradiol, and distilled water was added thereto, then the liquid remained in a suspended state. To the suspension was added γ-cyclodextrin, it became a transparent aqueous solution. This aqueous solution was made eye drops C. A total amount of the α- + γ-cyclodextrin was 24.6 mM (molar ratio; 6.7:1).

Eye drops D: eye drops of inclusion material of methyl-β-cyclodextrin and 17β-estradiol

In 15 mM of methyl-β-cyclodextrin was included 3.67 mM (1 mg/ml) of 17β-estradiol, and the product was dissolved in distilled water to prepare a transparent and uniform aqueous solution. This aqueous solution was made eye drops D (molar ratio; 4.09:1).

When 17β-estradiol was included in 2-hydroxypropyl-β-cyclodextrin, α- + γ-cyclodextrin or methyl-β-cyclodextrin, water-solubility is heightened and eye drops (eye drops B to D) which are transparent and stable can be prepared.

Example 2 Effects on keratoconjunctival tissue

For the purpose of investigating actions of eye drops B to D prepared in Example 1 on eyes, they are dropped

to rabbit, mouse and human being and evaluated.

When eye drops B (2-hydroxypropyl-β-cyclodextrin inclusion product) was dropped to eyes of house rabbit (Dutch rabbit) four times each with 50 μl (1 drop) with 30 minutes intervals, no specific problem occurred. Also, when the same was dropped to mouse once (50 μl) a day for 8 months and to human being with the same amount for 11 months, no specific problem occurred.

When eye drops C (α- + γ-cyclodextrin inclusion product) was dropped to eyes of human being, no stimulation occurred.

When eye drops D (methyl-β-cyclodextrin inclusion product) was dropped to eyes of house rabbit (Dutch rabbit) four times each with 50 μl (1 drop) with 30 minutes intervals:

after 30 minutes, a number of corneal erosion was increased,
and blood vessel around the cornea injected;
after 1 hour, eye discharge appears, and an area of corneal erosion enlarged;
after 1 hour and 30 minutes, a number of erosion becomes large and a depth of the same becomes deep; and at 2 hours, injection becomes remarkable, and corneal epithelium whole detachment and corneal ulcer occurred at peripheral portion of cornea with a shape of triangle having a base of 2 mm and a height of 3 mm so that the experiment was stopped.

Moreover, when eye drops D was dropped to human being with 1 drop (50 μl), strong stimulation occurred, lacrimation occurred, and a sense of incongruity continued for 5 hours till going to bed, but it recovered next morning.

Eye drops B and C, particularly eye drops B causes no specific problem even when it is dropped, and found out that it is optimum as eye drops. On the other hand, eye drops D has potent stimulation, changes physiologic chemical properties of cornea or conjunctiva, and causes injection, morbid epithelium detachment of cornea, and corneal ulcer, so that it can be found out that it is unsuitable as eye drops.

Example 3 Concentration of 17β-estradiol to transfer into anterior chamber of eye

To investigate effects of eye drops A to D prepared in Example 1, measurement of a concentration of the 17β-estradiol transferred into anterior chamber after dropping was carried out by using house rabbit (Dutch rabbit: female) with blind study.

At 10 A.M., each eye drops was dropped with 1 drop (50 μl), and after 5 minutes, further 1 drop was dropped. After dropping, at 30 minutes, 1 hour, 2 hours, and at 3 hours, cornea was subjected to centesis by using 27 gauge injection needle and 1 ml of a syringe to collect 0.1 ml of aqueous humor in anterior chamber to make a specimen. The specimen was subjected to C18 column extraction, and then, an amount of the 17β-estradiol was measured by using DPC double antibody estradiol kit (Diagnostic Products Corporation, USA). This is a method in which radioactivity is measured by a gamma counter using an estradiol antibody and an iodized estradiol $^{125}$I reagent. The measured values are calculated in terms of pg/ml. The results are shown in Fig. 3. A concentration of the 17β-estradiol in the anterior chamber immediately before dropping was 153 pg/ml in average.

As can be seen from Fig. 3, in either of the eye drops, a concentration of the 17β-estradiol in anterior chamber showed the highest value after 30 minutes from dropping, and thereafter decreased. Since the concentration of the 17β-estradiol included in 2-hydroxypropyl-β-cyclodextrin of eye drops B in anterior chamber is the highest value, so that it can be said that it passes through cornea and conjunctiva most potently. From this result, it can be estimated that 2-hydroxypropyl-β-cyclodextrin acts as an enhancer of 17β-estradiol passing through cornea and conjunctiva, i.e., acts to easily pass through cornea and conjunctiva. That which showed the secondly highest concentration of the 17β-estradiol in anterior chamber was eye drops C in which the 17β-estradiol was included in α- + γ-cyclodextrin. α-Dextrin has a low solubility of 17β-estradiol, and a function of γ-dextrin seems to be more potent. Eye drops A (suspension) was low transferred concentration into anterior chamber, and showed poor permeability. The reason why such a result was obtained can be considered that, in the eye drops A, it causes aggregated clogs with at random size and a particle size becomes large as one of the factors. Eye drops D in which the 17β-estradiol was included in methyl-β-cyclodextrin showed a higher transferred concentration of the 17β-estradiol in anterior chamber (the value after 30 minutes from dropping: 36178 pg/ml), but in this sample, physiologic chemical properties of cornea and conjunctiva have changed by methyl-β-cyclodextrin, and it was morbid permeation through pathologic cornea and conjunctiva, so that it was excluded from the graph.

From the experimental results, it was found that the eye drops B which is an inclusion product of 2-hydroxypropyl-β-cyclodextrin was the best.

Example 4 Treatment of cataract (animal experiment)

Animal experiments to investigate a treatment effect of cataract by 17β-estradiol eye drops (17β-estradiol concentration: 0.1 g/l and 0.01 g/l) prepared from an inclusion product of water-soluble 17β-estradiol in 2-hydroxypropyl-β-cyclodextrin available from SIGMA Co. (USA) were carried out with blind study.

Control group (Control): To eyes of mouse (4 eyes) were irradiated ultraviolet rays UV-A + UV-B (each 0.36J: 100 μW x 1 hour) from 10 PM to 11 PM at the first day, the third day, the fifth day each once, and three times in total. Provided that, before 1 minute and after 1 minute of each ultraviolet ray irradiation, 1 drop (50 μl) of PBS (phosphate buffered physiological saline) was dropped, and 1 drop of PBS was dropped each once at the second day, the fourth day, the sixth day, the seventh day, the eighth day, the ninth day and the tenth day.

Pre-administered group of ultraviolet ray irradiation (Pre 0.1 g/l and Pre 0.01 g/l): To eyes of mouse were irradiated ultraviolet rays UV-A + UV-B (each 0.36J: 100 μW x 1 hour) from 10 PM to 11 PM at the first day, the third day, the fifth day each once, and three times in total. Provided that before 1 minute of each ultraviolet ray irradiation, 1 drop (50 μl) of eye drops with a 17β-estradiol concentration of 0.1 g/l (4 eyes) or 0.01 g/l (4 eyes) was dropped, and after 1 minute of the ultraviolet ray irradiation, 1 drop of PBS was dropped, and at the second day, the fourth day, the sixth day, the seventh day, the eighth day, the ninth day and the tenth day, 1 drop (50 μl) of eye drops with a 17β-estradiol concentration of 0.1 g/l or 0.01 g/l was dropped each once.

Post-administered group of ultraviolet ray irradiation (Post 0.1 g/l and Post 0.01 g/l): To eyes of mouse were irradiated ultraviolet rays UV-A + UV-B (each 0.36J: 100 μW x 1 hour) from 10 PM to 11 PM at the first day, the third day and the fifth day each once, and three times in total. Provided that before 1 minute of each ultraviolet ray irradiation, 1 drop of PBS was dropped, and after 1 minute of the ultraviolet ray irradiation, 1 drop (50 μl) of eye drops with a 17β-estradiol concentration of 0.1 g/l (4 eyes) or 0.01 g/l (4 eyes) was dropped, and at the second day, the fourth day, the sixth day, the seventh day, the eighth day, the ninth day and the tenth day, 1 drop (50 μl) of eye drops with a 17β-estradiol concentration of 0.1 g/l or 0.01 g/l was dropped each once.

At 10 PM of the tenth day, an anterior portion of the eye was photographed (Fig. 4).

As can be seen from Fig. 4, when ultraviolet rays UV-A + UV-B were irradiated to eyes of the mouse for 3 days, at the tenth day, cataract appeared in Control group to which PBS alone was administered, while in the pre-administered group of irradiation and the post-administered group of irradiation to which 17β-estradiol-containing eye drops had been dropped, it could be confirmed that occurrence of cataract had scarcely observed. The administered group of eye drops with a higher concentration of 17β-estradiol showed less appearance of cataract than the administered group of eye drops with a lower concentration of the same, and the pharmaceutical effects were more potent. Also, the pre-administered group of ultraviolet ray irradiation showed less appearance of cataract than the post-administered group of irradiation, and the pharmaceutical effects appeared more potent. In 2-hydroxypropyl-β-cyclodextrin-included 17β estradiol, it could be confirmed that both of the prevention effect and the treatment effect of cataract by dropping had been present.

Eye drops containing 2-hydroxypropyl-β-cyclodextrin-included 17β-estradiol (17β-estradiol concentration 10 mg/l) were added to eyes of mouse and when nucleus of lens cortex cells were subjected to immune stain by an antibody of an estrogen receptor, peripheral of nucleus and in the nucleus were stained as described hereinbelow, whereby it could be confirmed that 17β-estradiol had been reached into the lens cortex cells.

Example 5 Transfer of estrogen receptor into nucleus of retinal external granular (nuclear) layer cells (Animal experiment)

To three-months old male mouse (Body weight: about 20g) were dropped 1 drop (50 μl) of eye drops with a 17β-estradiol concentration of 100 mg/l prepared as mentioned in Example 7 below once in the morning at the first day, twice in the morning and evening at the second day, and once in the morning at the third day. Cells (visual cells) at the retinal external granular (nuclear) layer of the retina of the mouse after dropping form 3 days were made samples. As a control, a sample from a mouse to which distilled water was dropped in the same manner was used.

According to the experimental method mentioned hereinbelow, the samples were subjected to immune stain using an anti-estrogen receptor clone AER311 (mouse monoclonal IgG 2aK) (200 μg/200 μl), and observed by a microscope (x100). The results are shown in Figs. 5 and 6.

In control, it could be confirmed that transfer of the estrogen receptor to the nucleus of the cells (visual cells) at the external granular (nuclear) layer of mouse retina had been a little (see Fig. 5), but in the cells (visual cells) at the external granular (nuclear) layer of mouse retina to which water-soluble 17β-estradiol-containing eye drops was dropped, it could be confirmed that the receptor was transferred into nucleus of the cells with a larger amount as compared to those of the control (see Fig. 6). According to the water-solubilized eye drops, it could be confirmed that the 17β-estradiol had been transferred even to a deep layer (retina) in the eye, and it could be proved that the objective cells are activated.

Example 6 Molecular biological experiment (Animal experiment)

It could be found that the 17β-estradiol can be sufficiently transferred into eyes according to dropping by including with 2-hydroxypropyl-β-cyclodextrin, so that detailed experiments were carried out molecular biologically.

(Experimental materials and method)

<Experimental materials>

As the mouse, C57BL/6 (purchased from Saitama Experimental Animals Supply Co., Ltd.) was used. For anesthesia at the time of dissection, ether was used.

Eye drops was prepared from 2-hydroxypropyl-β-cyclodextrin-included 17-β estradiol (SIGMA Co., MO, USA), and used with a 17-β estradiol concentration of 0.01 to 100 μg/ml.

Anti-estrogen receptor monoclonal antibody clone AER311 was purchased from Upstate Co. (NY, USA). A reagent necessary for immune dye containing a secondary antibody was purchased from VENTANA Co. (AZ, USA).

<Preparation method of ice-cold slice>

After passing a predetermined time from dropping, mouse was anesthetized with ether to euthanasia, an eyeball containing a partial optic nerve was extracted, and ice-cooled with PBS (phosphate buffered physiological saline) containing 2% paraformaldehyde to fix it for 1 hour. Then, on isopentane cooled with dry ice, it was embedded by using TISSU MOUNT (Shiraimatu Instrument Co., Ltd.). The slice was immersed in an HE fixing solution (79% ethanol, 20% formalin, 1% acetic acid) for 5 minutes for post-fixation using Cryostat (LEICA CM1900, GERMANY). It was washed with PBS and applied to stain scanning.

<Detection of estrogen receptor by immune stain>

A sliced piece was subjected to immune stain using an HX system of VENTANA Co. As stain protocol, DAB Non-Paraffin protocol was selected, the sample was subjected to antigen-activation treatment by a conditioner CCl, and reacted with an anti-estrogen receptor antibody diluted to a concentration of 4 μg/ml with an antibody diluting solution as a primary antibody for 32 minutes. Coloration using a secondary antibody and diaminobenzidine (DAB) was carried out by using Ventana Amplification Kit. Also, BLUING REAGENT was used for post-counterstain. After completion of stain, penetration was carried out by using ethanol and xylene, and mounting was carried out by using Soft Mount (Wako Junyaku K.K.).

<Observation of stained slice>

For observation of the slice subjected to immune stain, upright microscope was used, the image was photographed by a digital camera (Nikon CoolPix 900), and analyzed by displaying it on a personal computer using an image treatment soft Photoshop.

(Results)

<Distribution of estrogen receptor in eyeball>

As a function and mechanism of estrogen now has been clarified, there have been known activation of estrogen receptor in cells by binding to estrogen, transfer into nucleus, increment of transcription activity of a specific gene group by binding to an enhancer. Thus, it is examined where a receptor on which the estrogen is required to be acted exists in eyeball. As a result, when it was observed by coloration using DAB, anti-estrogen receptor antibody positive image could be obtained at retina containing an external granular (nuclear) layer (Fig. 7), part of lens (Fig. 8), cornea (Fig. 9), and ciliary body (Fig. 10). At ciliary body, background was strong so that judgment was difficult.

Confirmation was carried out whether 17β-estradiol was transferred into a deep portion of eye due to dropping, and it is effective or not for treatment of various kinds of eye ground diseases, optic nerve diseases such as glaucoma, etc.

<Transfer to nucleus of retinal estrogen receptor due to dropping of 17β-estradiol>

As mentioned above, the presence of an estrogen receptor was admitted in retina. However, as shown in Fig. 7, its localization was cytoplasm, and this state is an inactivated type. It has been reported with a large number in culture

cells, etc., that when estrogen binds, then transfer into nucleus occurs. However, in vivo, it is quite uncertain whether estrogen induces transfer of the receptor to nucleus, and this can be confirmed by immune stain of tissue cut piece or not. Thus, whether transfer of receptor of retina to nucleus can be admitted or not by dropping of estrogen was firstly examined.

(1) Transfer of estrogen receptor to nucleus in retina

To mouse was dropped 1 drop (50 μl) of eye drops containing estrogen (2-hydroxypropyl-β-cyclodextrin-included 17β-estradiol 10 μg/ml) four times of noon at the 1st day, morning and evening at the 2nd day, and morning at the 3rd day, and after 1 hour from the final dropping, eyeball was extracted, froze, and sliced, and then stained with an anti-estrogen receptor antibody. As a result, in retinal external granular (nuclear) layer cells, an image transferred into nucleus with a certain ratio can be confirmed (Fig. 11 to Fig. 14). Based on the results, in the following, change in dropped 17β-estradiol concentration and change due to a time after addition are to be analyzed.

(2) Change due to dropped 17β-estradiol concentration

For examining an dropped 17β-estradiol concentration which is capable of transferring an estrogen receptor of retinal external granular (nuclear layer) cells into nucleus, various concentrations of 2-hydroxypropyl-β-cyclodextrin-included 17β-estradiol was dropped four times, noon at the 1st day, morning and evening at the 2nd day, and morning at the 3rd day, in the same manner as in (1), and after 1 hour from the final dropping, eyeball was extracted, froze, and sliced, and then stained with an anti-estrogen receptor antibody. The results are shown in Fig. 15. When eye drops with a 17β-estradiol concentration of 0.01 μg/ml or more was dropped, transfer of the receptor into the nucleus can be meaningfully admitted as compared with eye drops (17β-estradiol 0 μg/ml) containing PBS (phosphate buffered physiological saline) alone. Transfer into nucleus increased up to 100 μg/ml of 17β-estradiol concentration dose-dependently.

(3) Change after dropping 17β-estradiol

For the purpose of examining until when transfer of an estrogen receptor into nucleus continues by the dropped 2-hydroxypropyl-β-cyclodextrin-included 17β-estradiol, 17β-estradiol with a concentration of 10 μg/ml was dropped three times with 10 minutes intervals, and eyeballs were extracted after 2, 8, 24 and 48 hours from the initial dropping, froze, and sliced, and then stained with an anti-estrogen receptor antibody. The results are shown in Fig. 16. As can be seen from this figure, after 2 hours from the dropping, transfer into nucleus can be seen in 36% of retinal external granular (nuclear layer) cells, it became 22% and 19% after 24 hours and 48 hours, respectively, and decrease in a number of transferred cells can be admitted until when slightly exceeding 18% of control in which 0 μg/ml of 17β-estradiol was added.

According to dropping of 17β-estradiol, it can be confirmed that, at retinal external granular (nuclear layer) cells, a receptor thereof is transferred into nucleus dose-dependently. These results clearly show that 17β-estradiol directly acts on a deep layer of eye by dropping. However, the effects decreased after 1 to 2 days. Also, the estrogen receptor transferred into nucleus is subjected to proteolysis.

(Conclusion)

An action of 2-hydroxypropyl-β-cyclodextrin-included 17β-estradiol on eyeball due to dropping was molecular biologically confirmed by using model animals. This is to show that estrogen such as 17β-estradiol, etc. can be easily acted on eyes by dropping using this method for clinical application with data.

When 0.01 μg/ml or more of 17β-estradiol was dropped, as compared with control in which PBS was dropped, transfer of an estrogen receptor in retinal external granular (nuclear layer) cells into nucleus can be meaningfully admitted, transfer into nucleus is dose-dependent with a concentration of from 0.1 μg/ml to up to 100 μg/ml, and it can be found out that a concentration of 0.1 μg/ml to 100 μg/ml or more is a suitable concentration of 17β-estradiol-containing eye drops.

Also, effects can be continued for a long time (24 hours or longer) from a time lapse of transfer into nucleus, so that it is sufficient with an administration of once or twice per 1 to 2 days, and it can be found that a number of administrations of eye drops can be made a little.

From the experiments, it can be expected that treatments of various kinds of eye ground diseases, treatment of optic nerve diseases such as glaucoma, etc., are possible by dropping, and it can be said that 2-hydroxypropyl-β-cyclodextrin-included 17β-estradiol-containing eye drops is an excellent dropping treatment agent for various kinds of diseases.

Example 7 Preparation of eye drops/eye washes

Water-soluble 17β-estradiol inclusion product (Cyclodextrin-encapsulated 17β-estradiol available from SIGMA CO. (USA); an inclusion product using 2-hydroxypropyl-β-cyclodextrin, and containing 45 mg of 17β-estradiol per 1 g of the inclusion product) was dissolved in sterilized distilled water with a concentration of 2.2 g/l (100 mg/l of 17β-estradiol) to prepare eye drops/eye washes.

Example 8 Effects on intraocular pressure (Animal experiment)

For the experiments, five (10 eyes) colored house rabbits (body weight: 1.8 to 2 kg) with 8 months to 10 months from the birth were used.

(1) Fluctuation of intraocular pressure during a day

Fluctuation of intraocular pressure during a day of house rabbits was measured. Average values were 14 mmHg at 9 AM, 12 mmHg at 1 PM, 12 mmHg at 3 PM and 16 mmHg at 6 PM.
Average intraocular pressure was 14±2 mmHg.

(2) Effect 1 of eye drops/eye washes

As can be seen from fluctuation of intraocular pressure of house rabbit during a day, intraocular pressure increases in the evening, so that eye drops/eye washes were administered at 1 PM and the effects were observed. Intraocular pressure of the house rabbit before treating with eye drops/eye washes was 14 mmHg at 1 PM.
To eyes of the house rabbit was dropped 25 μl (1 drop) of 17β-estradiol eye drops/eye washes prepared in Example 7 per one eye at 1 PM. When intraocular pressure of the dropped house rabbit was measured at 4:30 PM which is after 3.5 hours from the dropping, it was lowered to 10 mmHg, and also, the effect continued at least up to 6 PM which is after 5 hours from the dropping.
On the other hand, a house rabbit which had not been dropped was washed with 17β-estradiol eye drops/eye washes prepared in Example 7 at 1 PM. The maximum dropping of the intraocular pressure appeared after 50 minutes from washing. Lowering effects of intraocular pressure continued at least up to after 5 hours from washing.

(3) Effect 2 of eye drops/eye washes

Experiment was carried out by using a house rabbit in which 1 eye alone was high intraocular pressure. (This data were not used for calculation of an average.)
Intraocular pressure of the house rabbit at 5 PM before dropping was 24 mmHg. When the house rabbit was washed with 17β-estradiol eye drops/eye washes prepared in Example 7 at 5 PM, intraocular pressure was lowered up to 15 mmHg after 15 minutes from washing. This house rabbit was immediately washed again with the same eye drops/eye washes, intraocular pressure was lowered up to 13 mmHg after 45 minutes from washing.
The house rabbit subjected to eye drops/eye washes treatment was allowed to stand, and intraocular pressure was measured next day at 9 AM, it was 18 mmHg, and the effects of eye drops/eye washes at the previous day remained. To the house rabbit was dropped 25 μl (1 drop) of 17β-estradiol eye drops/eye washes prepared in Example 7, intraocular pressure was lowered up to 13 mmHg after 3 hours and 15 minutes from dropping. At this point, 25 μl of 17β-estradiol eye drops/eye washes prepared in Example 7 was again dropped, intraocular pressure after 3 hours and 15 minutes from dropping again was 13 mmHg. Thereafter, 25 μl of the same eye drops/eye washes was further dropped, intraocular pressure was 12 mmHg after 1 hour and 50 minutes from the dropping.
Intraocular pressure at the next day (at the third day) before dropping was 10 mmHg at 9 AM, and at this point, 25 μl of 17β-estradiol eye drops/eye washes prepared in Example 7 was once dropped, intraocular pressure at noon (after 3 hours from dropping) was 9 mmHg, intraocular pressure at 3:15 PM was 12 mmHg, and intraocular pressure at 6 PM was 11 mmHg. The effects of dropping can be found to be continued for a long time.
Since the effects remain at the next day of the administration, a receptor of estrogen may pertain to and an action with a level of DNA may pertain to. There is a possibility due to generation of NO. Other mechanism than the above can be considered.

Example 9 Treatment effect of normal tension glaucoma patient

The patient is 75 years-old female diagnosed as normal tension glaucoma.
Water-soluble 17β-estradiol inclusion product (Cyclodextrin-encapsulated 17β-estradiol available from SIGMA

CO. (USA); an inclusion product using 2-hydroxypropyl-β-cyclodextrin, and containing 45 mg of 17β-estradiol per 1 g of the inclusion product) was dissolved in sterilized distilled water with a concentration of 2.2 g/l (100 mg/l of 17β-estradiol) to prepare eye drops.

To the above-mentioned patient, 1 drop (25 μl) of eye drops was dropped per one eye once every morning for 10 days.

Intraocular pressures of the patient were 14 mmHg right and 12 mmHg left before dropping, and were 12 mmHg right and 9 mmHg left after dropping for 10 days.

According to visual field measurement by Octopus 1-2-3 of the patient before dropping, visual field defect was confirmed (Figs. 17 and 18), but according to dropping for 10 days, both eyes extremely well improved (Figs. 19 and 20). Treatment effects of normal tension glaucoma can be clinically confirmed.

Example 10 Treatment effect of high intraocular pressure open-angle glaucoma

The patient is 54 years-old female diagnosed as high intraocular pressure open-angle glaucoma.

Water-soluble 17β-estradiol inclusion product (Cyclodextrin-encapsulated 17β-estradiol available from SIGMA CO. (USA); an inclusion product using 2-hydroxypropyl-β-cyclodextrin, and containing 45 mg of 17β-estradiol per 1 g of the inclusion product) was dissolved in sterilized distilled water with a concentration of 2.2 mg/l (100 μg/l of 17β-estradiol) to prepare eye drops.

To the above-mentioned patient, 1 drop (25 μl) of eye drops was dropped once every morning for a week.

Intraocular pressures of the patient were 23 mmHg right and 24 mmHg left before dropping, and were 16 mmHg right and 18 mmHg left after dropping for a week.

As for visual field, there was admitted scotoma, but in right eye, slight defect (Fig. 21) could be substantially normally recovered (Fig. 23), and in left eye, progressed defect (Fig. 22) could slightly improved (Fig. 24).

When dropping was further continued, at 3 weeks, intraocular pressures of the patient were increased to 20 mmHg in right and 19 mmHg in left before dropping. 100 μg/l of 17β-estradiol concentration is considered to be near to the lower limit of the concentration which reveals an effect. More effective treatment can be carried out by making a higher concentration, or increasing a number of times of dropping per 1 day.

Treatment effects of high intraocular pressure open-angle glaucoma could be clinically confirmed.

Example 11 Treatment of cataract (Clinical treatment effect)

A patient of cataract was treated by preparing eye drops with a 17β-estradiol concentration of 1 mg/l which had been prepared by dissolving water-soluble 17β-estradiol inclusion product used in Example 7 in sterilized distilled water, and dropped 1 drop (50 μl) thereof 8 AM every morning once per 1 day, then, improvement in visual acuity was admitted (see Fig. 25). Also, in a slit lamp microscope for examination, it was observed that opacity of cataract had been reduced.

Example 12 Treatment effect of presbyopia

The patient is 59 years-old male of presbyopia having slight myopia of -2.5 dioptre and -1.00 of astigmatism.

When a near point of the left eye of the patient was measured without lens correction, it was 29cm.

Water-soluble 17β-estradiol inclusion product (Cyclodextrin-encapsulated 17β-estradiol available from SIGMA CO. (USA); an inclusion product using 2-hydroxypropyl-β-cyclodextrin, and containing 45 mg of 17β-estradiol per 1 g of the inclusion product) was dissolved in sterilized distilled water with a concentration of 2.2 g/l (100 mg/l of 17β-estradiol) to prepare eye drops.

To the patient was dropped 1 drop (25 μl) of eye drops at 9 AM per each eye. The near point of the patient which had been 29 cm before dropping became 27 cm after 1 hour from the dropping, 24 cm after 3 hours, and improvement in refraction at the maximum of 5 cm was observed (Fig. 26). Incidentally, the far point was 41±2 cm in average.

Treatment effect of presbyopia by dropping was clinically proved.

Example 13 Treatment of pigmentary retinal degeneration

The patient is 86 years-old female, and has artificial intraocular lens inserted eyes already carried out a cataract operation. Due to pigmentary retinal degeneration, narrowing of visual field occurs. To the patient was dropped 1 drop (50 μl) of eye drops containing 17β-estradiol prepared in Example 7 in a concentration of 100 mg/l once everyday (8 AM), then, visual fields were gradually improved. After 77 days from initiation of dropping, it could be confirmed that both of visual acuity and visual field were improved.

Visual acuity before dropping was Vd=0.4xIOL (vision after correction: 0.6) and

Vs=0.8xIOL (vision after correction: 0.8)

But after 77 days, they were

Vd=0.4xIOL (vision after correction: 0.8)

Vs=0.5xIOL (vision after correction: 1.0)

whereby improvement in visual acuities could be confirmed.

Also, when the visual fields were measured by OCTOPUS 1-2-3 static perimeter before dropping and after 77 days from the initiation of the dropping, and they were compared, it can be confirmed to be improved. (see Figs. 27 to 30) Example 14 Treatment of chalazion

To right eye of 8 years-old girl with chalazion was dropped 1 drop (50 μl) of eye drops containing 17β-estradiol prepared in Example 11 with a concentration of 1 mg/l once every morning, then, tumor disappeared after 12 days. Example 15 Treatment of myopia

1. To 13 years-old boy [visual acuity:

Vd=0.7(1.5x-0.5D)

Vs=0.6(1.5x-0.5D)]

was dropped 1 drop (50 μl) of eye-drops prepared by dissolving water-soluble 17β-estradiol inclusion product used in Example 7 was dissolved in distilled water containing 17β-estradiol with a concentration of 100 μg/l once per day (8 AM), then, visual acuity was improved to

Vd=1.0(1.5x-0.25D)

Vs=1.0(1.5x-0.25D)

after 9 days.

2. 13 years-old boy [visual acuity:

Vd=0.4(1.5x-0.75D)

Vs=0.3(1.5x-1.25D)]

was dropped 1 drop (50 μl) of eye-drops prepared in Example 11 containing 17μ-estradiol with a concentration of 1 mg/l once every morning, then, visual acuity was improved after 45 days from dropping as mentioned below.

Vd=1.0 (1.5x-0.5D)

Vs=0.5 (1.5x-1.0D)

Example 16 Treatment of presbyopia

To 49 years-old male with presbyopia having corrected near visual acuity (near vision after correction) before treatment of

Vd=0.6/30 cm x NJB

Vs=0.4/30 cm x NJB

was dropped 1 drop (50 μl) of eye drops prepared in Example 11 containing 17μ-estradiol with a concentration of 1 mg/l once every morning, then, after 14 days of dropping, near vision after correction was improved as mentioned below.

Vd=1.0/30 cm x NJB

Vs=0.7/30 cm x NJB

Example 17 Treatment of chorioretinal hemorrhage

To 80 years-old male having chorioretinal hemorrhage of the right eye was dropped 1 drop (50 μl) of eye drops prepared in Example 11 containing 17β-estradiol with a concentration of 1 mg/l once every morning, and simultaneously total amount of 30 mg of Adona (trade name) was orally administered three times a day, then, hemorrhage disappeared after 45 days.

Example 18 Treatment of corneal ulcer

To right eye of 76 years-old female who is a patient having recurrent corneal ulcer at the both eyes alternately and diagnosed as Mooren's corneal ulcer was dropped 1 drop (50 μl) of eye drops containing 17β-estradiol prepared in Example 11 with a concentration of 1 mg/l twice a day at 8 AM and 5 PM, then the disease was cured for 3 weeks. Thus, it can be considered that treatment of autoimmune disease is possible.

Example 19 Treatment of corneal degeneration

1. To left eye of 63 years-old female diagnosed as granular corneal degeneration was dropped 1 drop (50 μl) of 17β-estradiol eye drops with a concentration of 1 mg/l prepared in Example 11 twice (8 AM and 3 PM) a day, then visual acuity was improved for 2 weeks.

   Vs=0.6 (n·c)→Vs=0.4 (0.8)

2. To right eye of 68 years-old female diagnosed as granular corneal degeneration was dropped 1 drop (50 μl) of the same eye drops as mentioned above once every morning, then improvement in visual acuity was observed for 1 week as Vd=0.3 (0.9)→Vd=0.3 (1.0), and after 2 months, it was markedly improved as Vd=0.5 (1.2). The value in the parentheses shows vision after correction.

Example 20 Treatment of iridocyclitis

To a 85 years-old female patient the right eye of whom observed iridocyclitis was dropped 1 drop (50 μl) of eye drops with a 17β-estradiol concentration of 1 mg/l prepared in Example 11 at 8 AM every morning, then the disease was lightened after 1 week (inflammatory cells in anterior chamber disappeared).

Example 21 Treatment of Posner-Schlossmans' Syndrome

To a 36 years-old patient left eye of whom observed iritis and increase in intraocular pressure (Tos=36 mmHg) diagnosed as Posner-Schlossmans' Syndrome was dropped 1 drop (50 μl) of eye drops with a 17β-estradiol concentration of 1 mg/l and prepared in Example 11 once at 8 AM every morning a day for 1 week, then iritis disappeared and intraocular pressure was also normalized (Tos=17 mmHg).

Example 22 Treatment of central nerve diseases

To 80 years-old female diagnosed as schizophrenia was treated by preparing eye drops with a 17β-estradiol concentration of 1 mg/l which had been prepared by dissolving water-soluble 17β-estradiol inclusion product used in Example 7 in sterilized distilled water, and dropped 1 drop (50 μl) thereof every morning once per 1 day, then, as mentioned below, improvement in visual acuity and improvement in mental state were obtained.

| | | |
|---|---|---|
| At the time of initiation | | Vd=0.1xIOL(0.3)<br>Vs=0.09xIOL(0.1) |
| | ↓ | 1 mg/l dropped with 1 drop (50 μl) once every morning |
| After 14 days | | Vd=0.1xIOL(0.4)<br>Vs=0.1xIOL(0.1)<br>The patient said that visual acuity was improved well. |
| | ↓ | 1 mg/l dropped with 1 drop (50 μl) once every morning |
| After 42 days | | Vd=0.15xIOL (0.4)<br>Vs=0.09xIOL (0.1)<br>The patient said that she felt to be encouraged. |
| | ↓ | 1 mg/l dropped with 1 drop (50 μl) once every morning |
| After 77 days | | Vd=0.3xIOL (0.6)<br>Vs=0.1xIOL (0.15) |
| | ↓ | 1 mg/l twice (50 μl) |
| After 115 days | | Vd=0.2xIOL (0.4)<br>Vs=0.09xIOL (0.2) |

The value in the parentheses shows vision after correction.

It could be confirmed that it is effective for treatment of a schizophrenia patient. It can be considered to be effective for treatments of other central nerve diseases, for example, Alzheimer's diseases, Parkinson's disease, ALS, psychosis, schizophrenia, manic-depressive psychosis, etc.

Example 23 Treatment of chorioretinal atrophy

To right eye of 65 year-old male was administered 50 μl of eye drops containing 1 mg/l of 17β-estradiol prepared in Example 11 once a day for 1 month, 0.08 (n·c) of visual acuity (impossible of correction) was improved to vision after correction of 0.2, and when left eye of 70 years-old female was administered 1 drop (50 μl) of the above-mentioned eye drops once a day for 1 month, vision after correction of 0.1 was improved to vision after correction of 0.3.

Example 24 Treatment of diabetic retinopathy

To a 74 years-old female patient having diabetic retinopathy was administered 1 drop (50 μl) of eye drops containing 1 mg/l of 17β-estradiol prepared in Example 11 through dropping once everyday. After about 2 months from initiation of the administration, retinal bleeding was observed by a photograph of eyegrounds, it was slightly improved. After about 100 days from initiation of the administration, retinal bleeding was disappeared.

Example 25 Treatment of macular hole

To left eye of 76 years-old female in which macular hole is being generated, and visual acuity of 0.7 was continuously dropped 1 drop (50 μl) of eye drops (10 mg/l) containing 2-hydroxypropyl-β-cyclodextrin-included 17β-estradiol twice a day for 1 month, then macular hole was cured, and visual acuity was recovered to 1.2.

Example 26 Improved effect in dynamic visual acuity

To left eye of 60 years-old male with vision after correction of Vs=0.1 (2.0 x-2.75 D ◯ -0.50D Ax 115°) was dropped 50 μl (1 drop) of eye drops (10 μg/ml 17β-estradiol concentration) containing 2-hydroxypropyl-β-cyclodextrin-included 17β-estradiol one time, and measurement of dynamic visual acuity was carried out by using dynamic visual acuity meter KV-100 manufactured by NIDEK. Dynamic visual acuity during 30 minutes to 1 hour and 15 minutes after dropping was abruptly improved. (Fig. 31)

Example 27 Regeneration and protection of corneal endothelial cells

To 30 years-old female patient whose endothelial cells were lost and decreased due to wearing contact lens was dropped 1 drop (50 μl) of eye drops containing 17β-estradiol (1 mg/l) prepared in Example 11, then it was improved for 10 days (dropped once a day).

Number of right eye endothelial cells (per $mm^2$): 2801 →3412

Number of left eye endothelial cells (per $mm^2$): 2906 →3496

As a measurement device, NONCON ROBO (KONAN INC) specular microscope was used.

Example 28 Improvement in photopsia

To 62 years-old female who complained photopsia was dropped 1 drop (50 μl) of eye drops (17β-estradiol concentration of 10 mg/l (10 μg/ml)) containing 2-hydroxypropyl-β-cyclodextrin-included 17β-estradiol once a day for 3 weeks, then the symptom was lightened.

Example 29 Treatment of epidemic keratoconjunctivitis

To 26 years-old female suffered from epidemic keratoconjunctivitis was administered 1 drop (50 μl) of eye drops (17β-estradiol concentration of 1 mg/l) containing 2-hydroxypropyl-β-cyclodextrin-included 17β-estradiol once a day, then keratitis superficialis diffusa was cured at the fourth day whereas eye discharge was increased.

Example 30 Treatment of asthenopia

To 30 years-old male who complained eye fatigue due to computer inputting operation was administered eye drops (17β-estradiol concentration of 10 mg/l) containing 2-hydroxypropyl-β-cyclodextrin-included 17β-estradiol once (50 μl) a day, then asthenopia was lightened for 3 weeks. Example 31 Treatment of central chorioretinopathy (chorioretinitis)

To 18 years-old male patient having visual acuity lowering in left eye and also having central chorioretinopathy (chorioretinitis) was dropped 1 drop (50 μl) of eye drops containing 2-hydroxypropyl-β-cyclodextrin-included 17β-estradiol (10 mg/l) twice a day, then visual acuity was improved for a week, and macular edema was lightened.

Visual acuity before dropping: Vs=0.1 (0.8x-2.5D ◯ cyl-2.75D Ax10°)

Visual acuity after treatment: Vs=0.3 (1.5x-2.5D ◯ cyl-2.25D Ax175°)

Utilizability in industry

According to the present invention, side effects which are problems of HRT can be overcome, and it is possible to provide a treating agent having high treatment effects of eye diseases such as glaucoma or cataract, etc., or a treating agent of central nerve diseases or psychosis.

Also, according to the present invention, it can be provided a treating agent of eye diseases such as glaucoma, cataract, eyegrounds diseases, etc., in which a time during which effects are continued is long, and is sufficient with one a day or once per 2 to 3 days. It can reduce intraocular pressure, and also improve narrowing of visual field particularly

markedly, so that not only a treatment of high tension glaucoma, but also a treatment of normal tension glaucoma in which no effective treatment method is not present as of today can be particularly effectively carried out.

## Claims

1. An agent which comprises sexual steroid hormone, a substance having estrogen action or selective estrogen receptor modulator, or non-feminizing estrogen for use in a method of treating cataract.

2. The agent for use according to Claim 1, wherein the sexual steroid hormone is selected from the group consisting of estrogen, testosterone, progesterone, androgen and their derivatives and metabolites.

3. The agent for use according to Claim 1 or 2, wherein the sexual steroid hormone is 17β-estradiol or its derivatives or metabolites.

4. The agent for use according to any one of Claims 1 to 3, wherein the sexual steroid hormone is a sexual steroid hormone solubilized in water.

5. The agent for use according to any one of Claims 1 to 4, wherein the sexual steroid hormone is a sexual steroid hormone included in cyclodextrin.

6. The agent for use according to any one of Claims 1 to 5, wherein the sexual steroid hormone is a sexual steroid hormone included in hydroxypropyl-β-cyclodextrin, hydroxyethyl-β-cyclodextrin, sulfobutyl ether-β-cyclodextrin, sulfonyl-β-cyclodextrin, γ-cyclodextrin, δ-cyclodextrin, or hydroxypropyl-γ-cyclodextrin.

7. The agent for use according to any one of Claims 1 to 6, wherein the sexual steroid hormone is a sexual steroid hormone included by 2-hydroxypropyl-β-cyclodextrin.

8. The agent for use according to Claim 1, wherein the substance having an estrogen action or selective estrogen receptor modulator, or non-feminizing estrogen is included in hydroxypropyl-β-cyclodextrin, hydroxyethyl-β-cyclodextrin, sulfobutyl ether-β-cyclodextrin, sulfonyl-β-cyclodextrin, γ-cyclodextrin, δ-cyclodextrin, or hydroxypropyl-γ-cyclodextrin, particularly in hydroxypropyl-β-cyclodextrin to solubilize in water.

9. The agent for use according to any one of Claims 1 to 8, wherein the agent is in a form of eye drops, eye washes, ointments, conjunctiva injections or contact lens adsorbents.

10. The agent for use according to any one of Claims 1 to 9, wherein the cataract is selected from senile cataract, cataract due to removal of overay and cataract due to TGF β.

Fig. 1

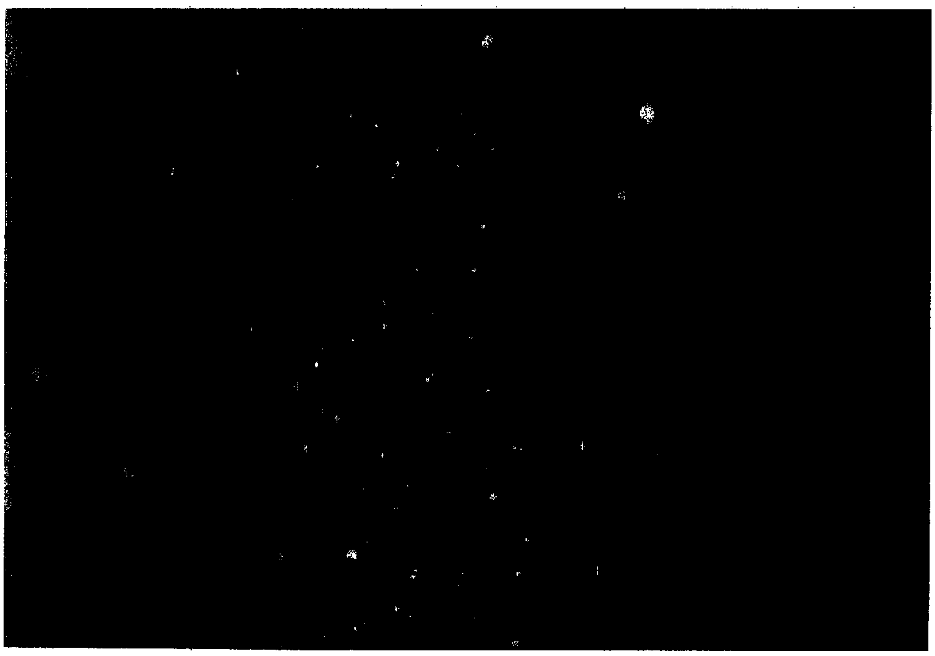

Fig. 2

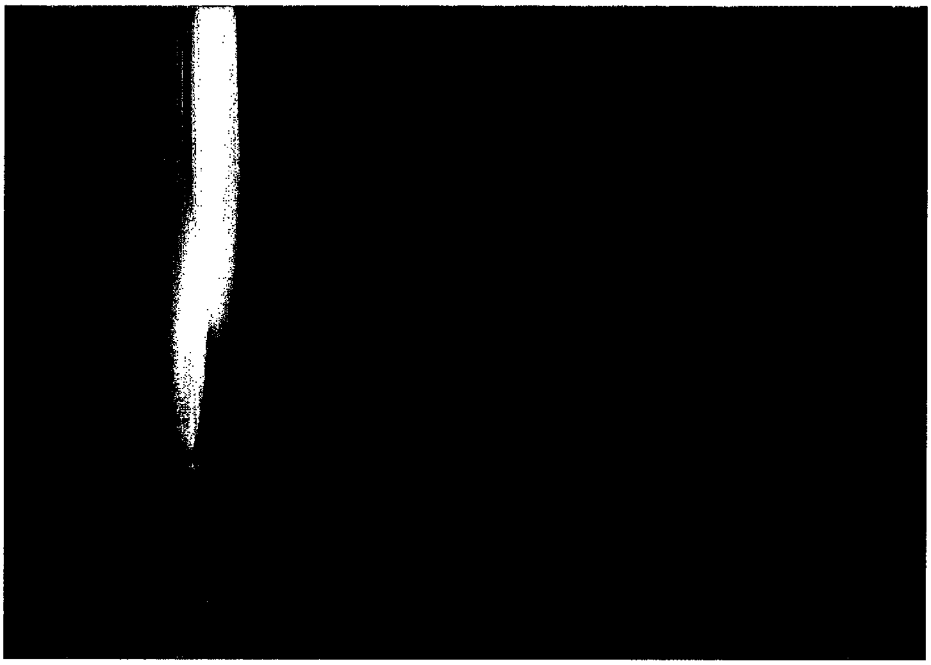

# Fig. 3

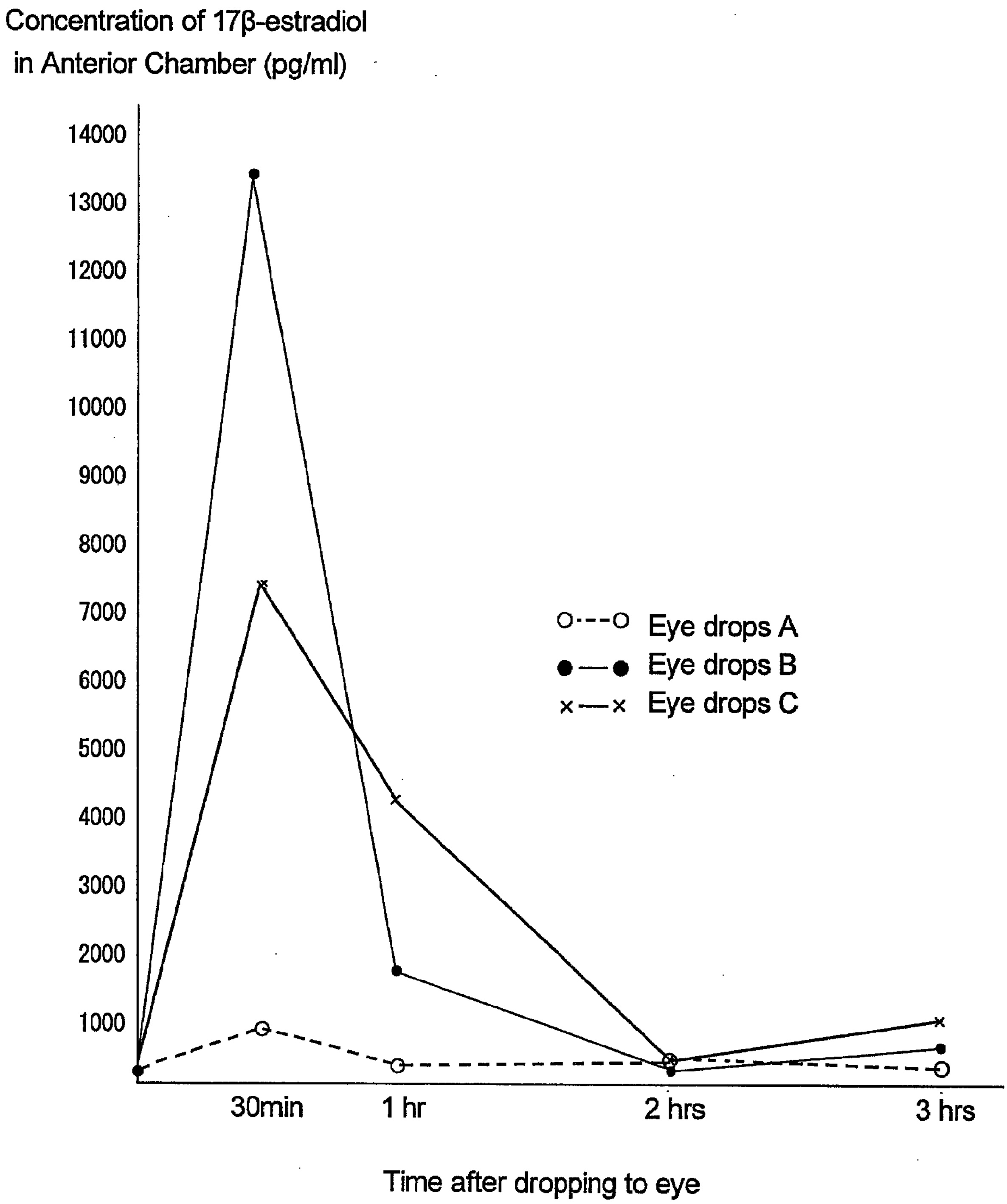
Concentration of 17β-estradiol
in Anterior Chamber (pg/ml)
14000
13000
12000
11000
10000
9000
8000
7000
6000
5000
4000
3000
2000
1000
Eye drops A
Eye drops B
Eye drops C
30min
1 hr
2 hrs
3 hrs
Time after dropping to eye

Fig. 4

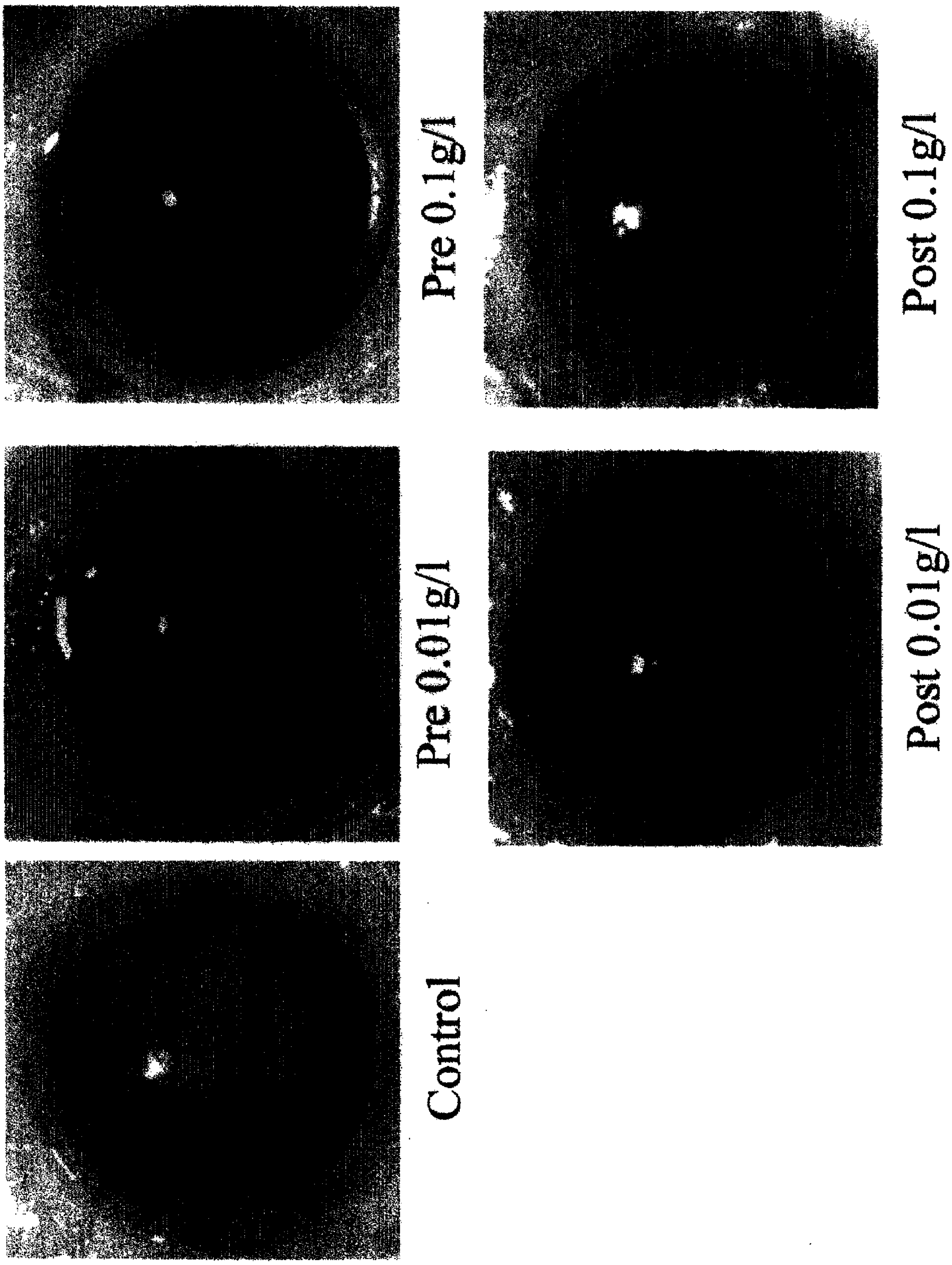

# Fig. 5

Retina of mouse eye dropped with distilled water
(external granular (nuclear) layer)

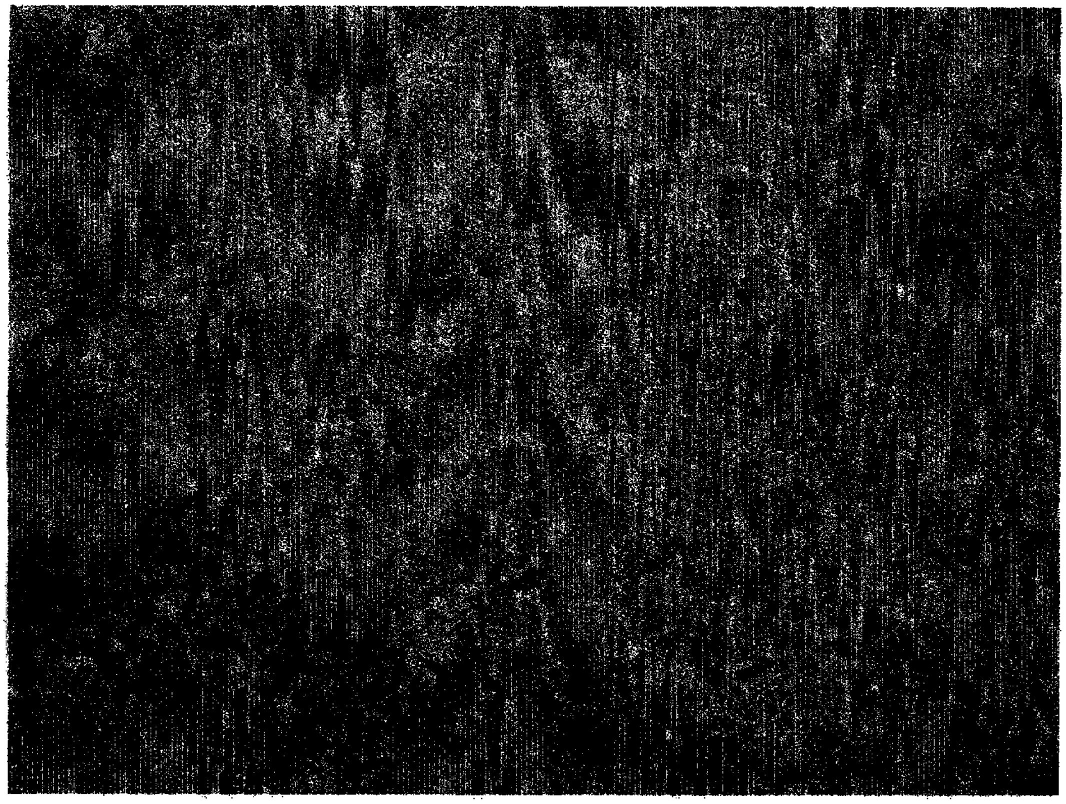

# Fig. 6

Retina of mouse eye dropped with estrogen
(external granular (nuclear) layer)

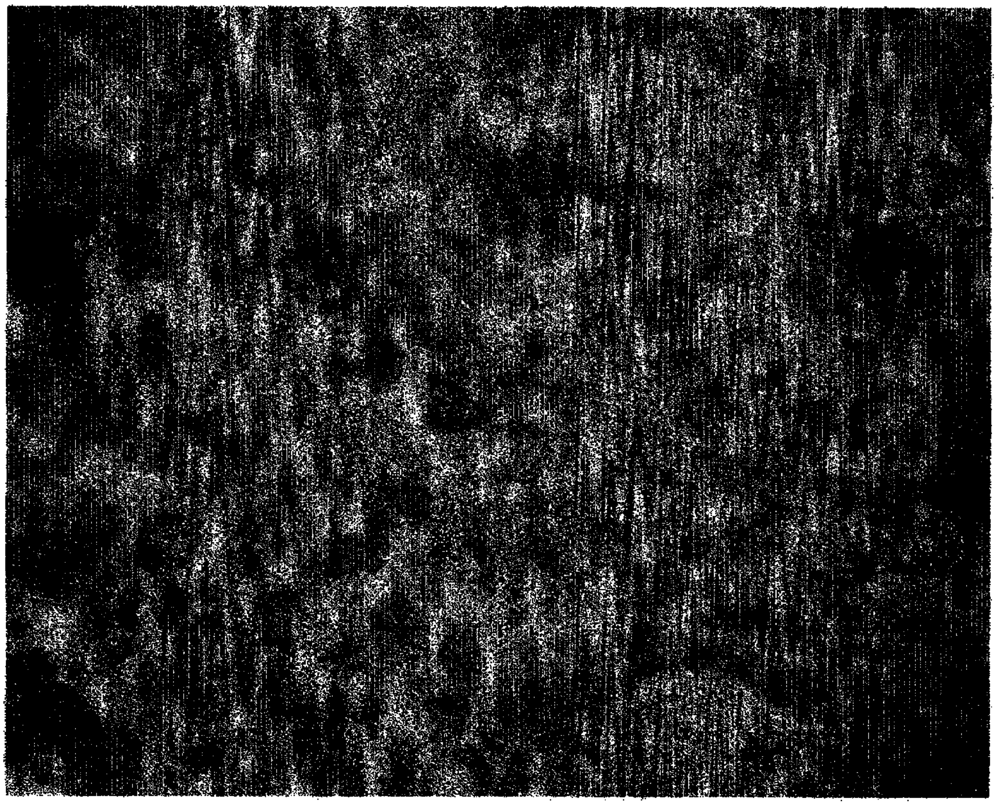

Fig. 7

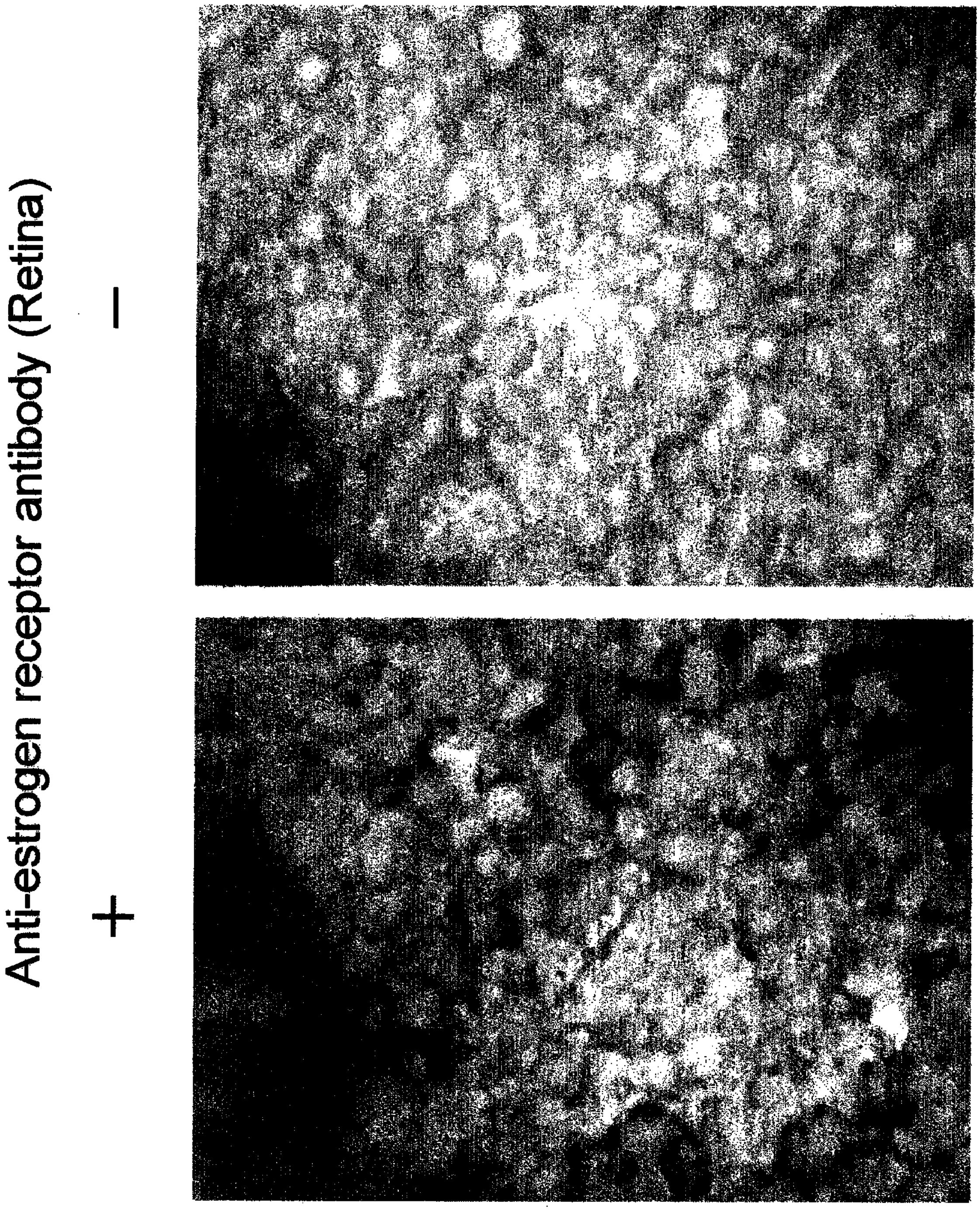

Fig. 8

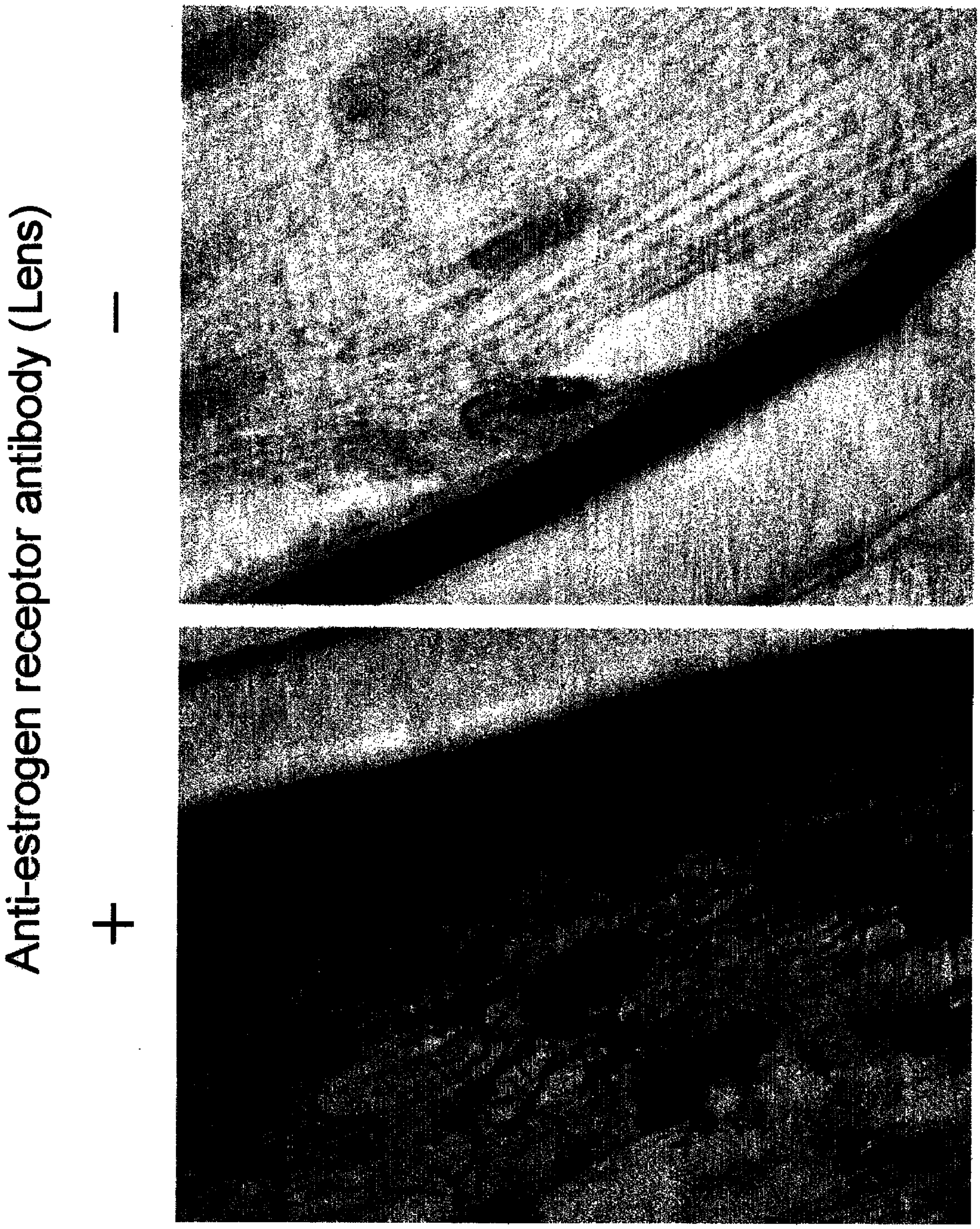

Fig. 9

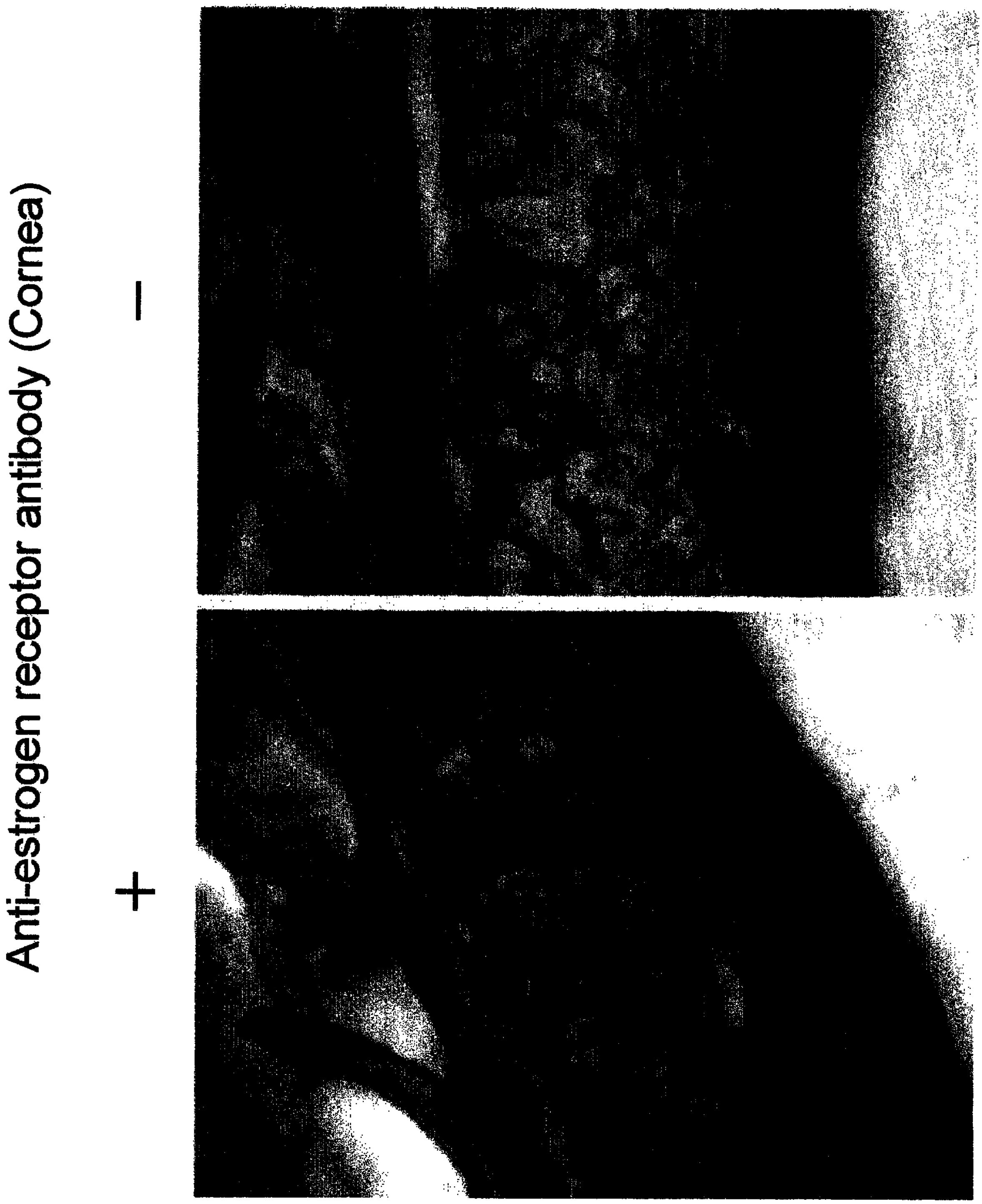

Fig. 10

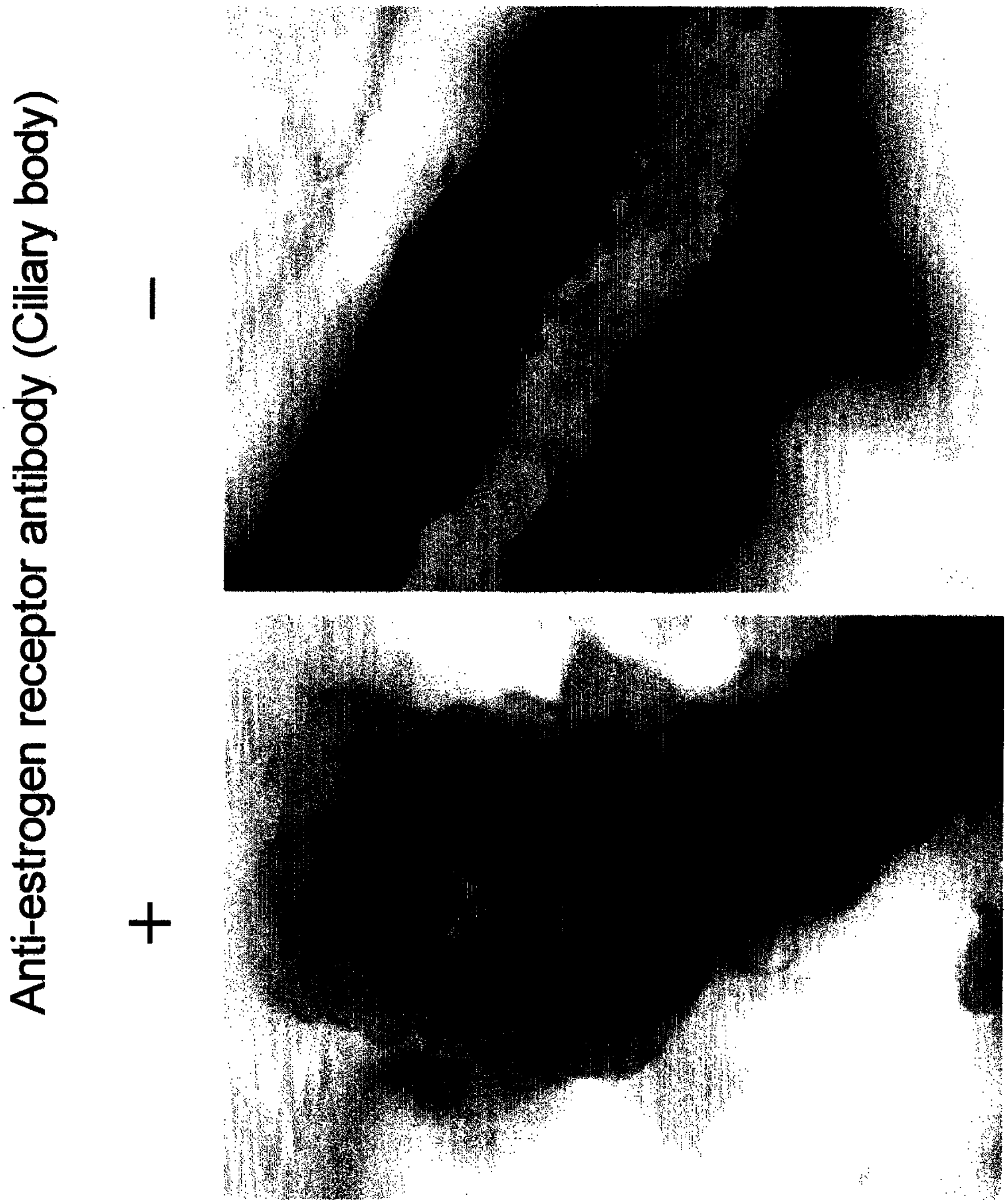

# Fig. 11

## Estrogen not added, antibody not added

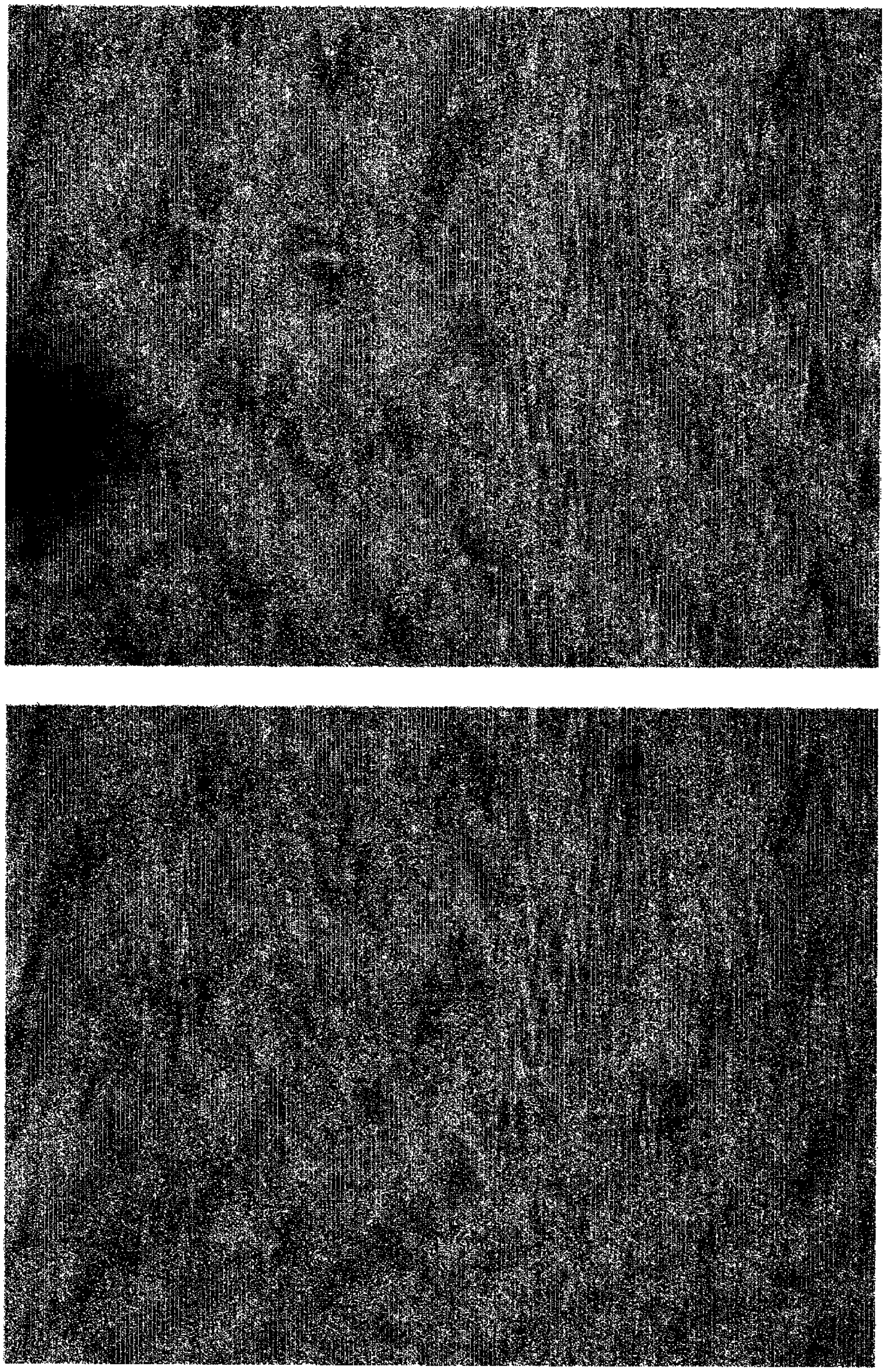

# Fig. 12

Estrogen not added, antibody added

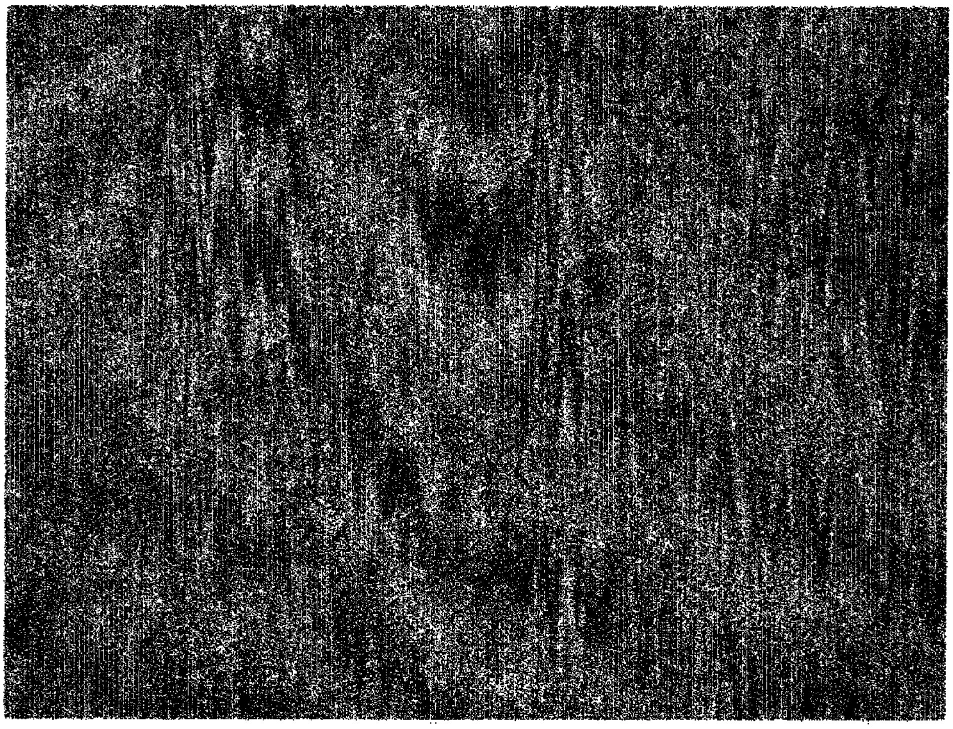

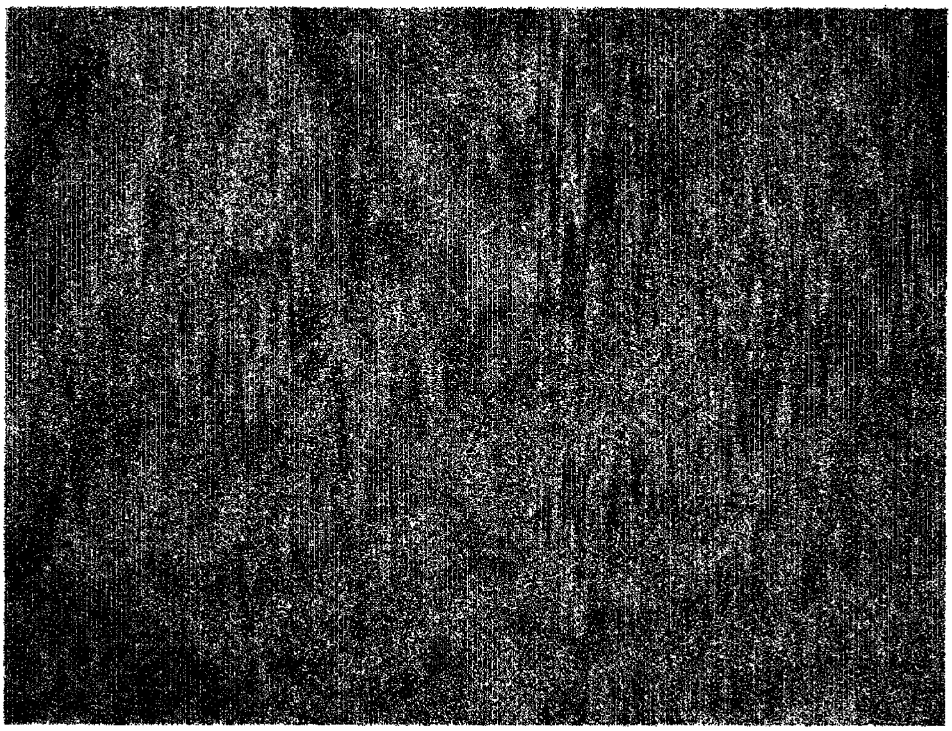

# Fig. 13

Estrogen added, antibody not added

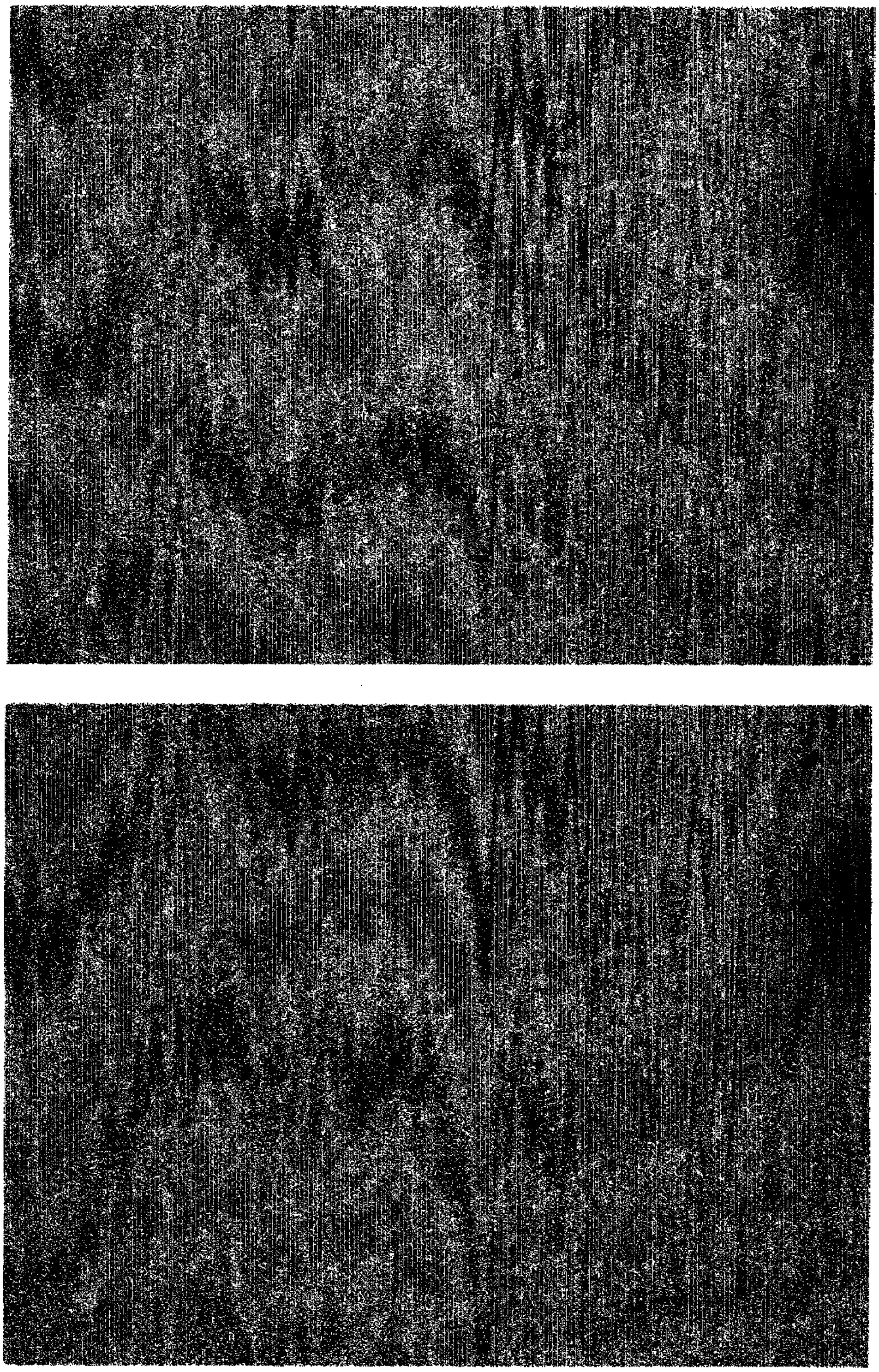

# Fig. 14

Estrogen added, antibody added

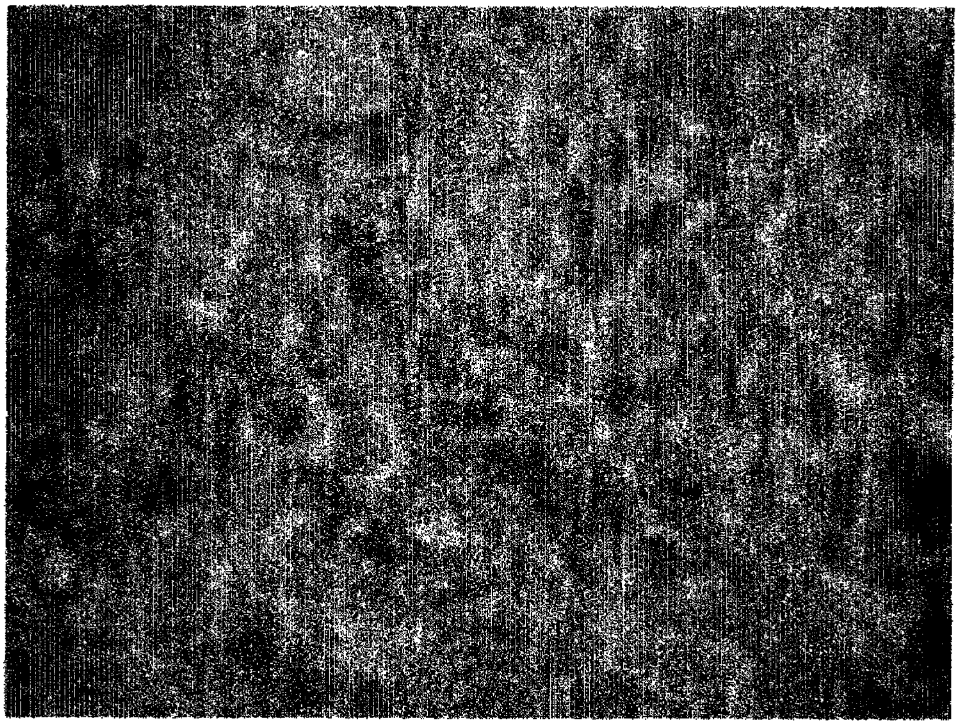

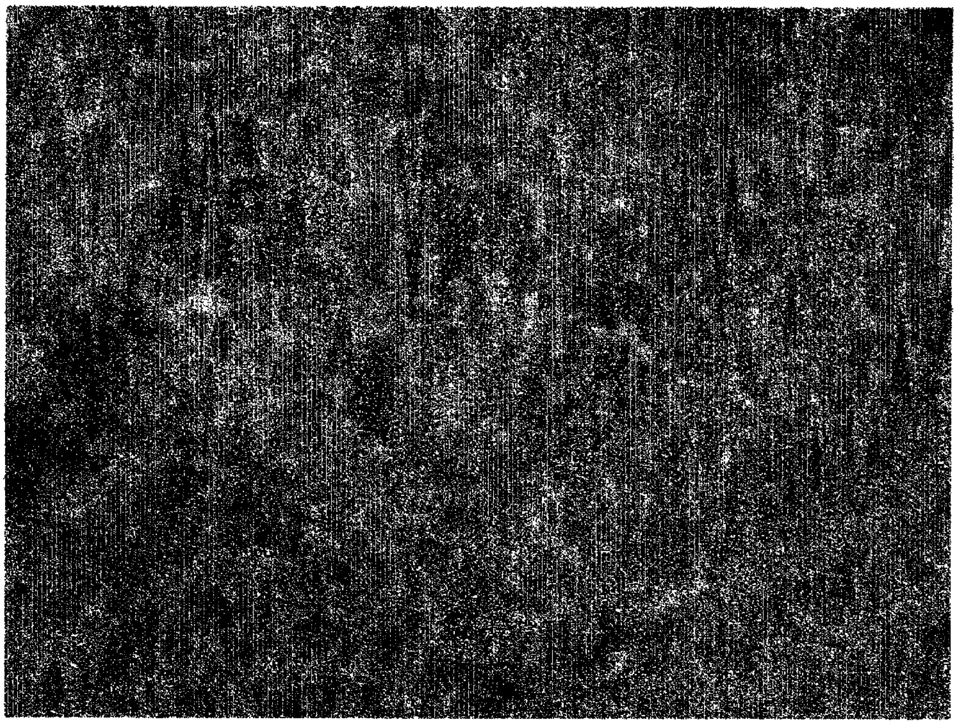

Fig. 15

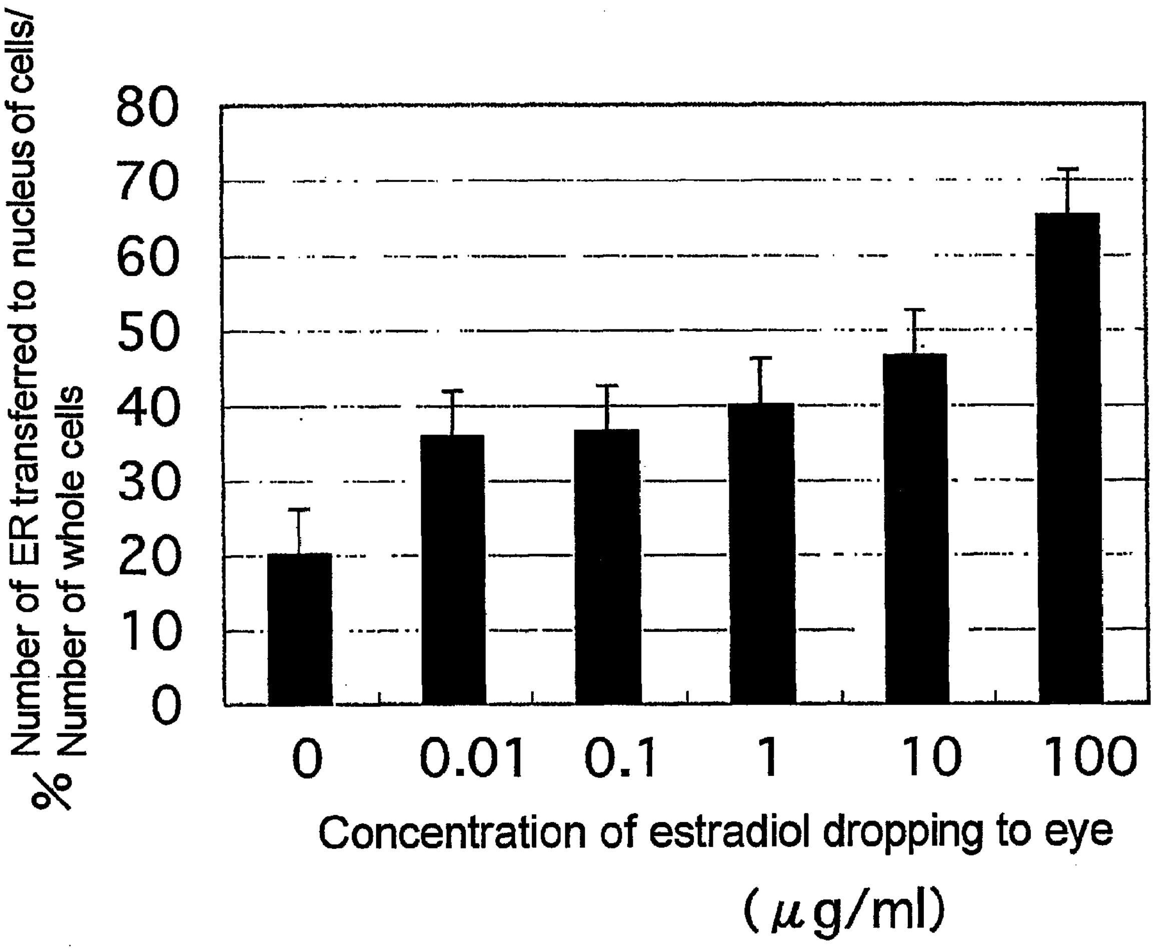
Number of ER transferred to nucleus of cells/
Number of whole cells
%
80
70
60
50
40
30
20
10
0
0
0.01
0.1
1
10
100
Concentration of estradiol dropping to eye
(μg/ml)

Fig. 16

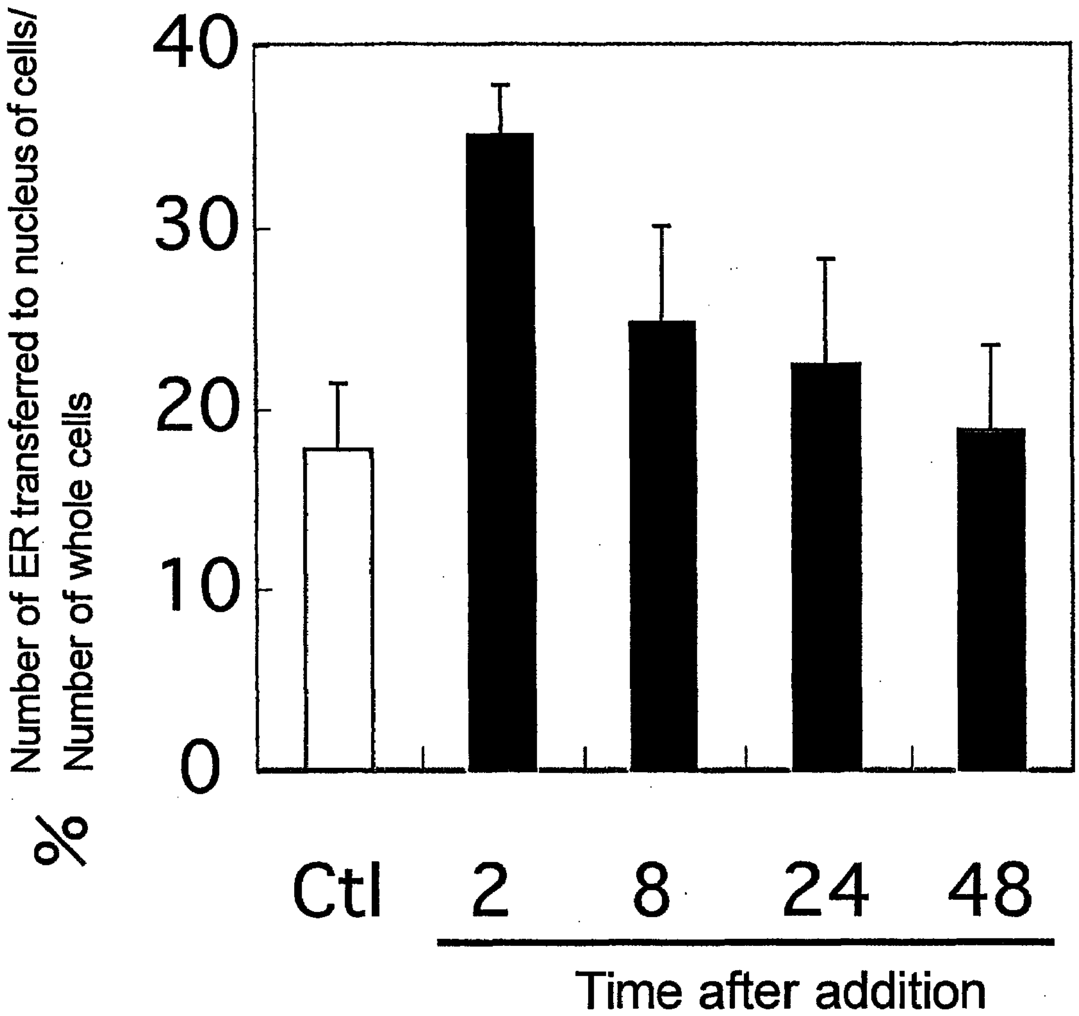

% Number of ER transferred to nucleus of cells/ Number of whole cells
40
30
20
10
0
Ctl
2
8
24
48
Time after addition

# Fig. 17

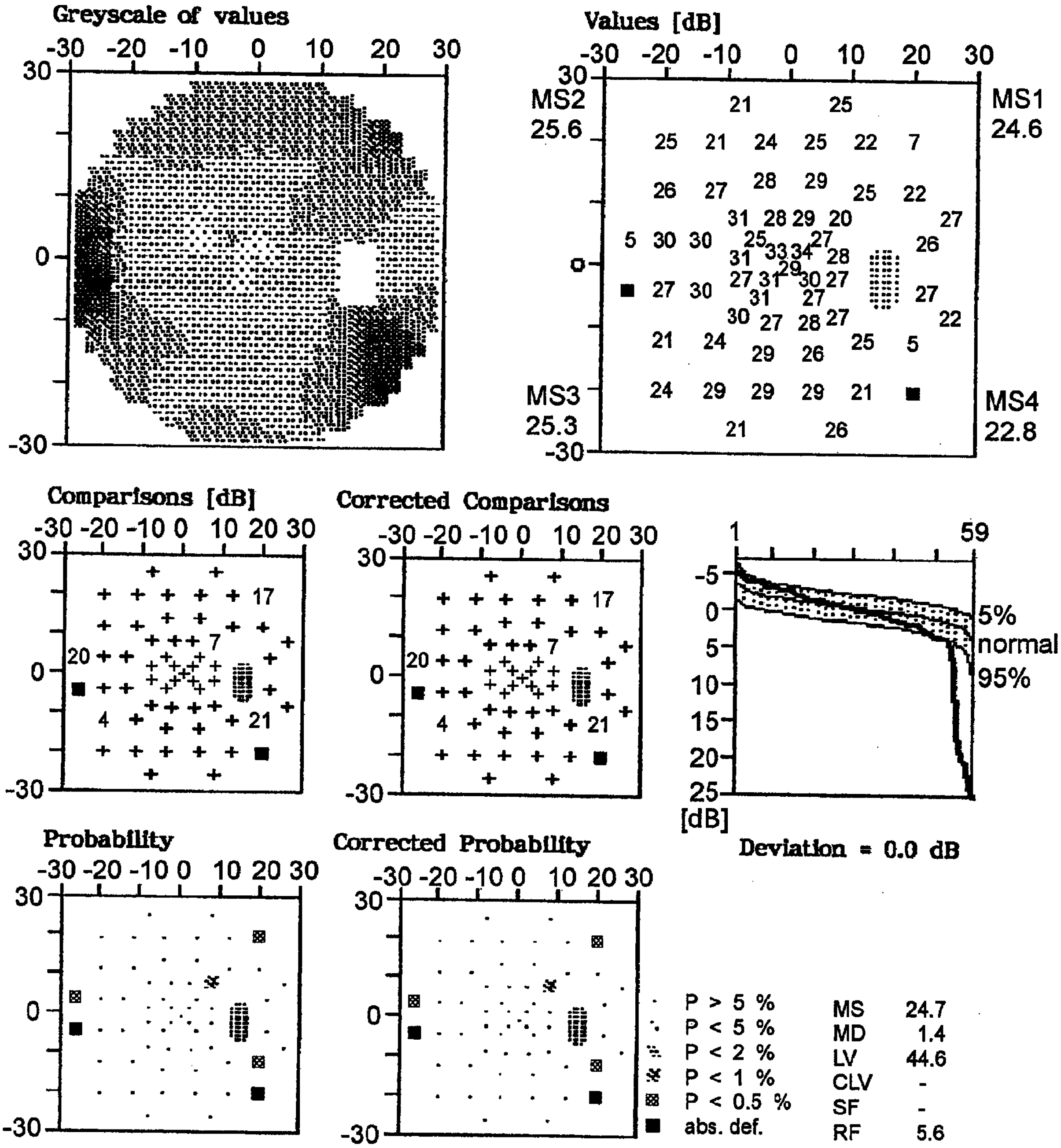

Greyscale of values
Values [dB]
MS1
24.6
MS2
25.6
MS3
25.3
MS4
22.8
Comparisons [dB]
Corrected Comparisons
5%
normal
95%
[dB]
Deviation = 0.0 dB
Probability
Corrected Probability
P > 5 %
P < 5 %
P < 2 %
P < 1 %
P < 0.5 %
abs. def.
MS 24.7
MD 1.4
LV 44.6
CLV -
SF -
RF 5.6

# Fig. 18

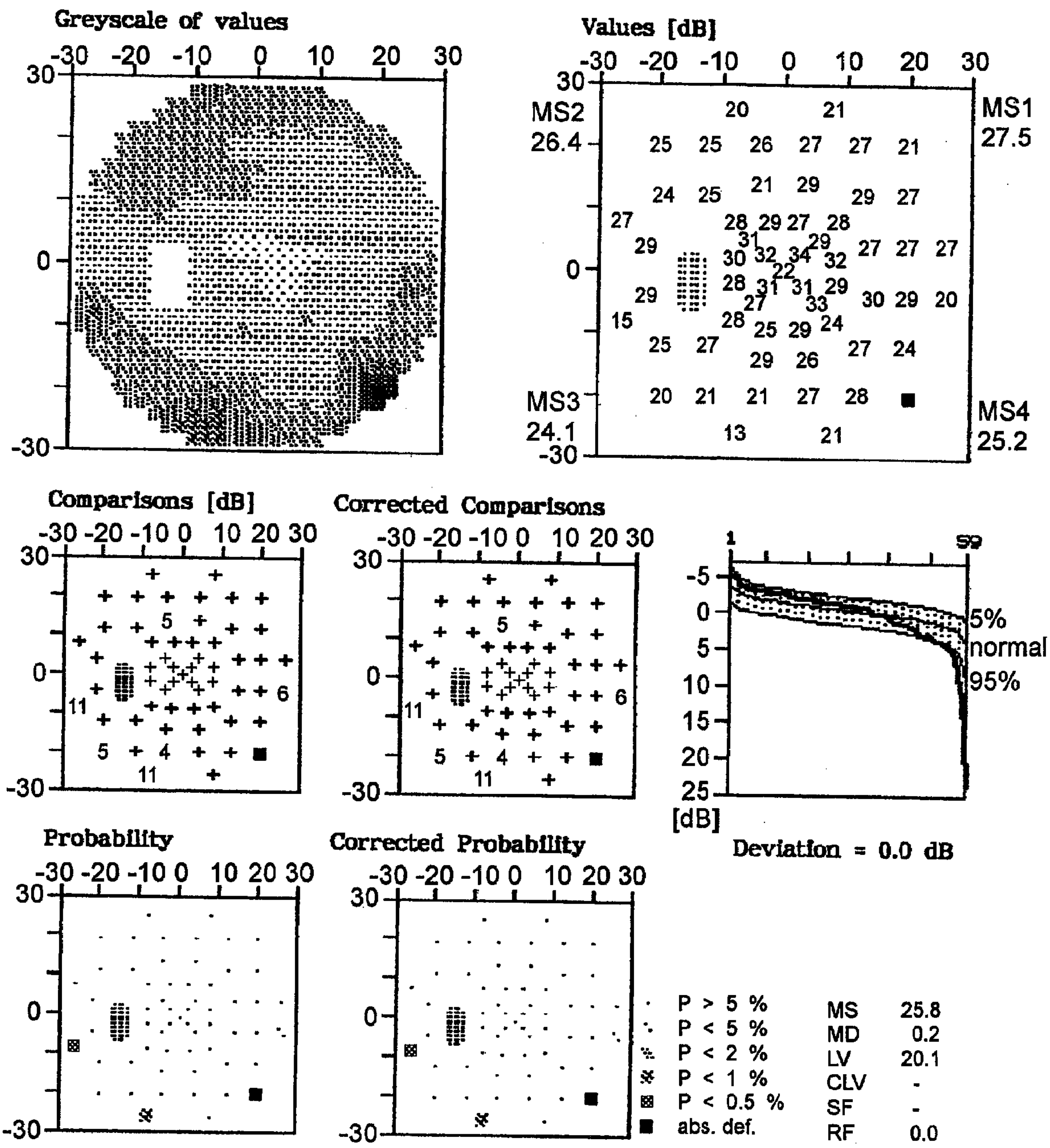
Greyscale of values
Values [dB]
MS2
26.4
MS1
27.5
MS3
24.1
MS4
25.2
Comparisons [dB]
Corrected Comparisons
5%
normal
95%
[dB]
Deviation = 0.0 dB
Probability
Corrected Probability
P > 5 %
P < 5 %
P < 2 %
P < 1 %
P < 0.5 %
abs. def.
MS 25.8
MD 0.2
LV 20.1
CLV -
SF -
RF 0.0

# Fig. 19

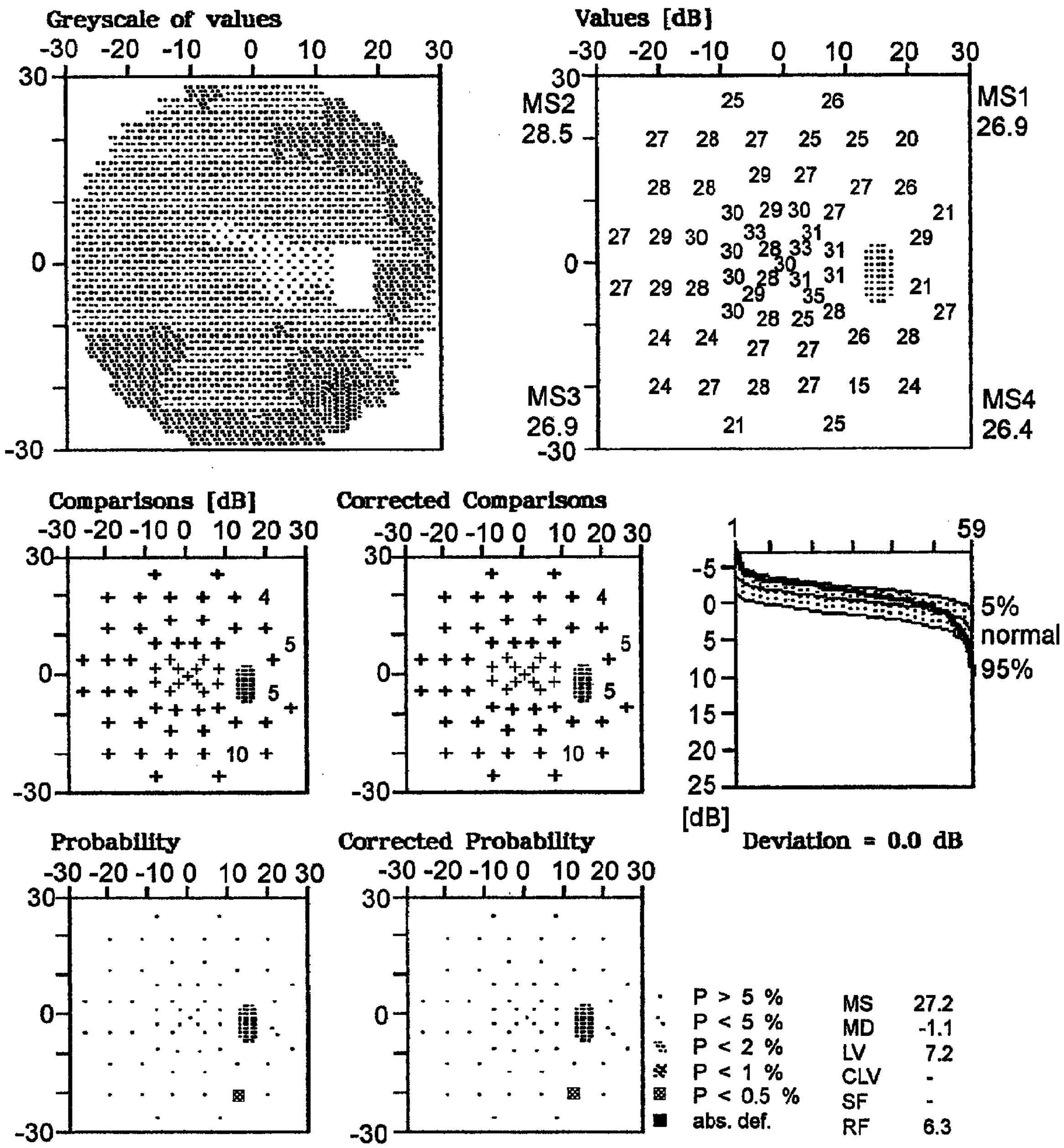
Greyscale of values
Values [dB]
MS1
26.9
MS2
28.5
MS3
26.9
MS4
26.4
Comparisons [dB]
Corrected Comparisons
5%
normal
95%
[dB]
Deviation = 0.0 dB
Probability
Corrected Probability
P > 5 %
P < 5 %
P < 2 %
P < 1 %
P < 0.5 %
abs. def.
MS 27.2
MD -1.1
LV 7.2
CLV -
SF -
RF 6.3

# Fig. 20

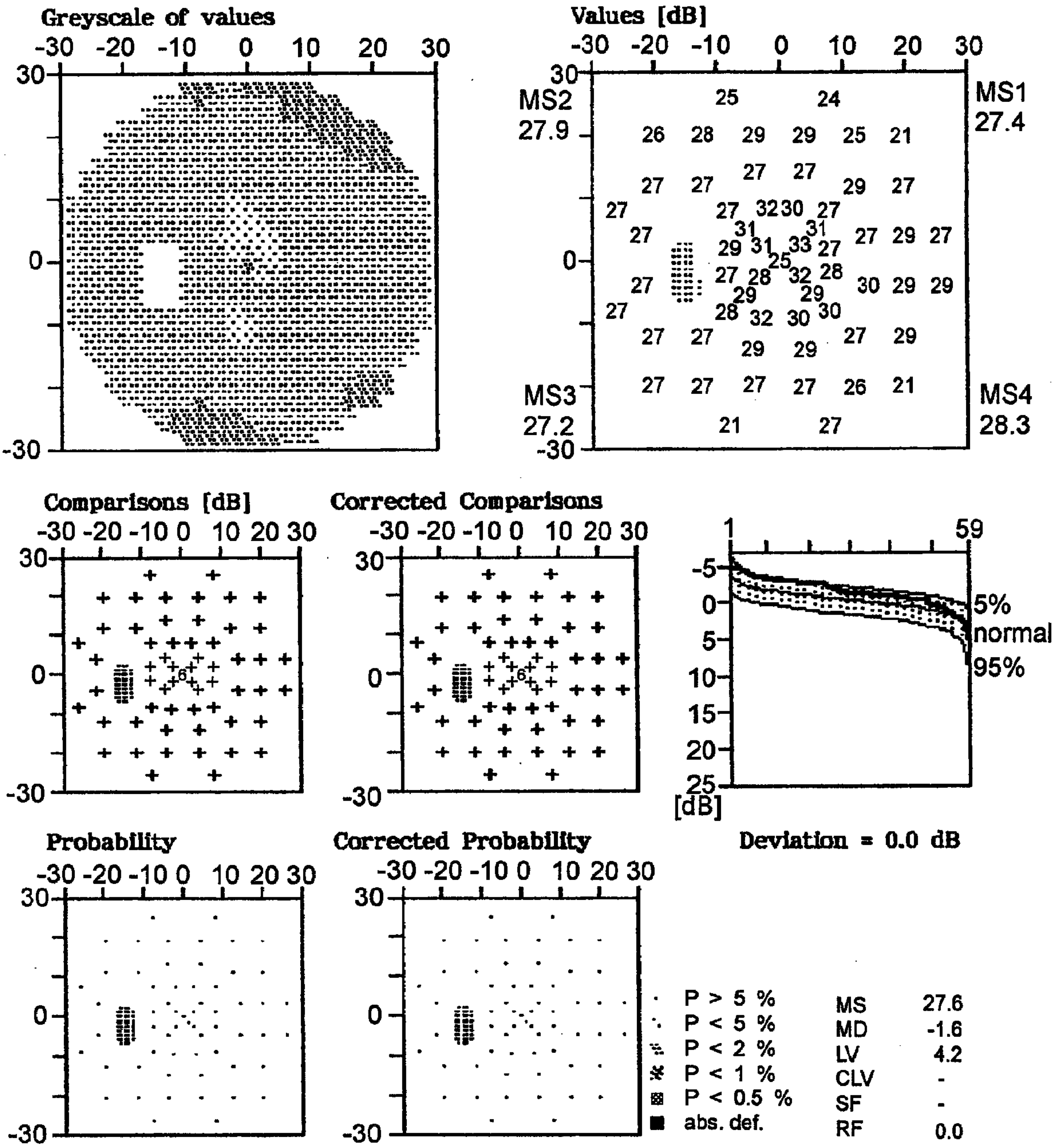
Greyscale of values
Values [dB]
MS1
27.4
MS2
27.9
MS3
27.2
MS4
28.3
Comparisons [dB]
Corrected Comparisons
5%
normal
95%
[dB]
Deviation = 0.0 dB
Probability
Corrected Probability
P > 5 %
P < 5 %
P < 2 %
P < 1 %
P < 0.5 %
abs. def.
MS 27.6
MD -1.6
LV 4.2
CLV -
SF -
RF 0.0

# Fig. 21

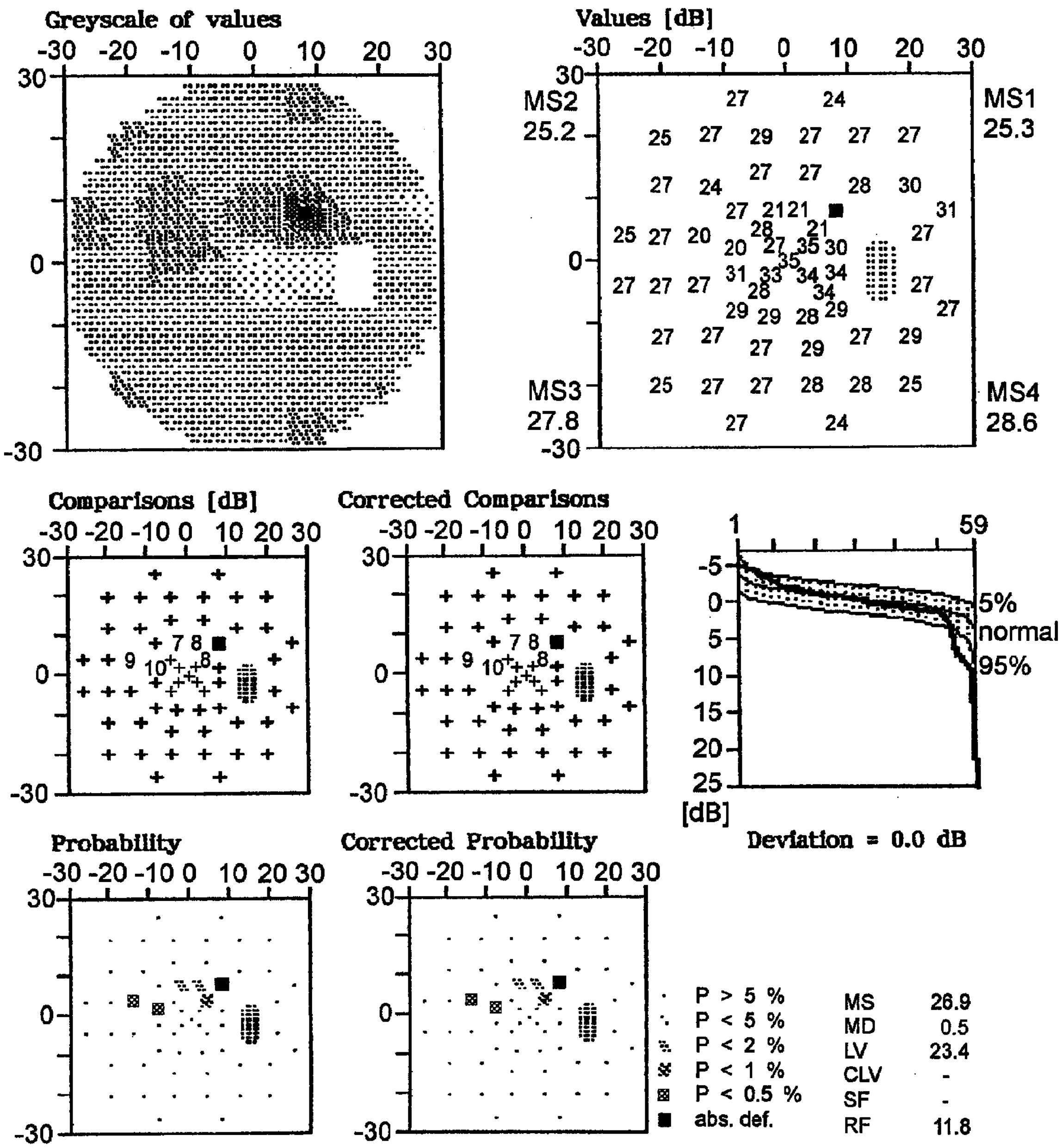
Greyscale of values
Values [dB]
MS1 25.3
MS2 25.2
MS3 27.8
MS4 28.6
Comparisons [dB]
Corrected Comparisons
5%
normal
95%
[dB]
Deviation = 0.0 dB
Probability
Corrected Probability
P > 5 %
P < 5 %
P < 2 %
P < 1 %
P < 0.5 %
abs. def.
MS 26.9
MD 0.5
LV 23.4
CLV -
SF -
RF 11.8

# Fig. 22

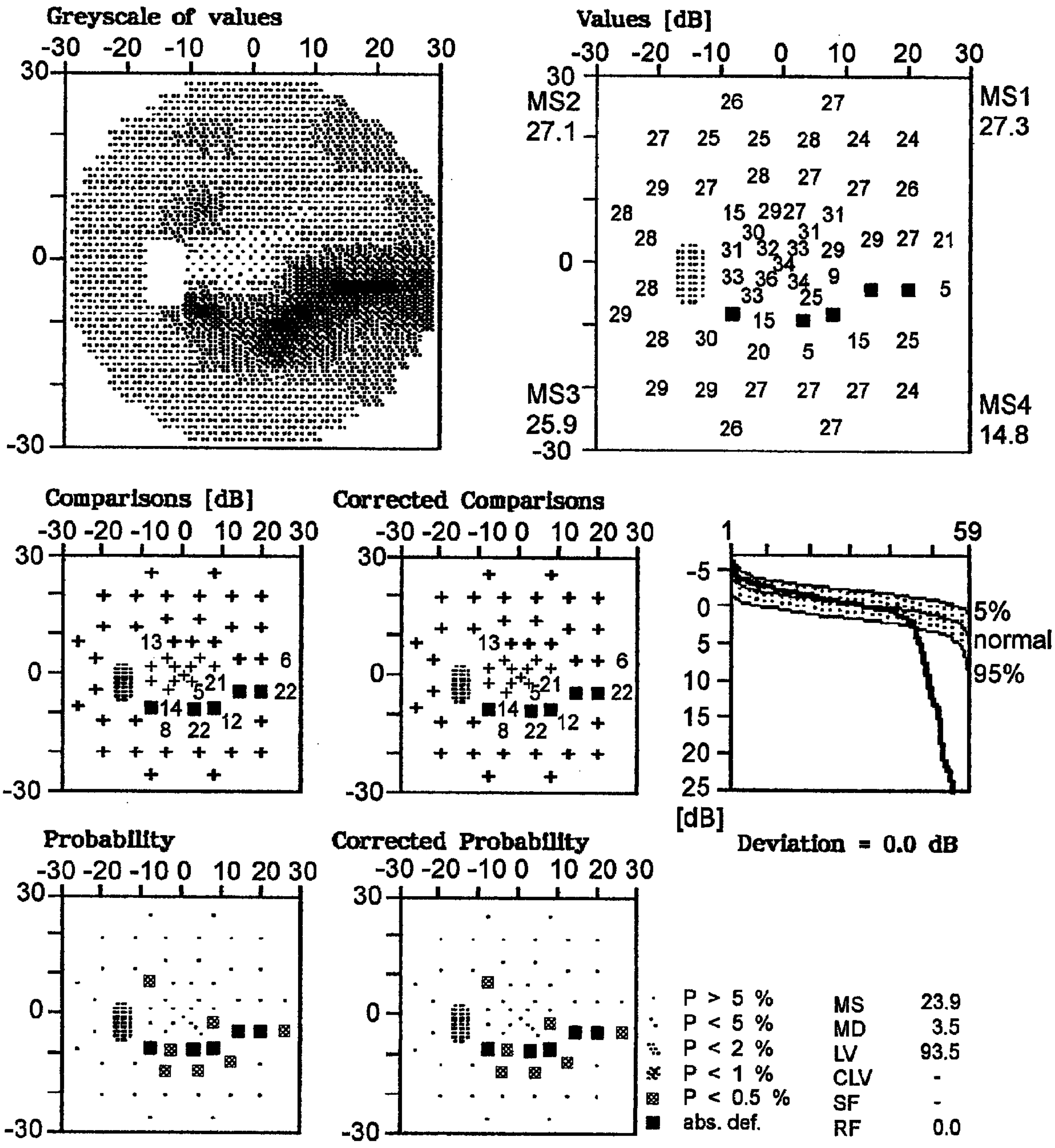
Greyscale of values
Values [dB]
MS1 27.3
MS2 27.1
MS3 25.9
MS4 14.8
Comparisons [dB]
Corrected Comparisons
5%
normal
95%
[dB]
Deviation = 0.0 dB
Probability
Corrected Probability
P > 5 %
P < 5 %
P < 2 %
P < 1 %
P < 0.5 %
abs. def.
MS 23.9
MD 3.5
LV 93.5
CLV -
SF -
RF 0.0

# Fig. 23

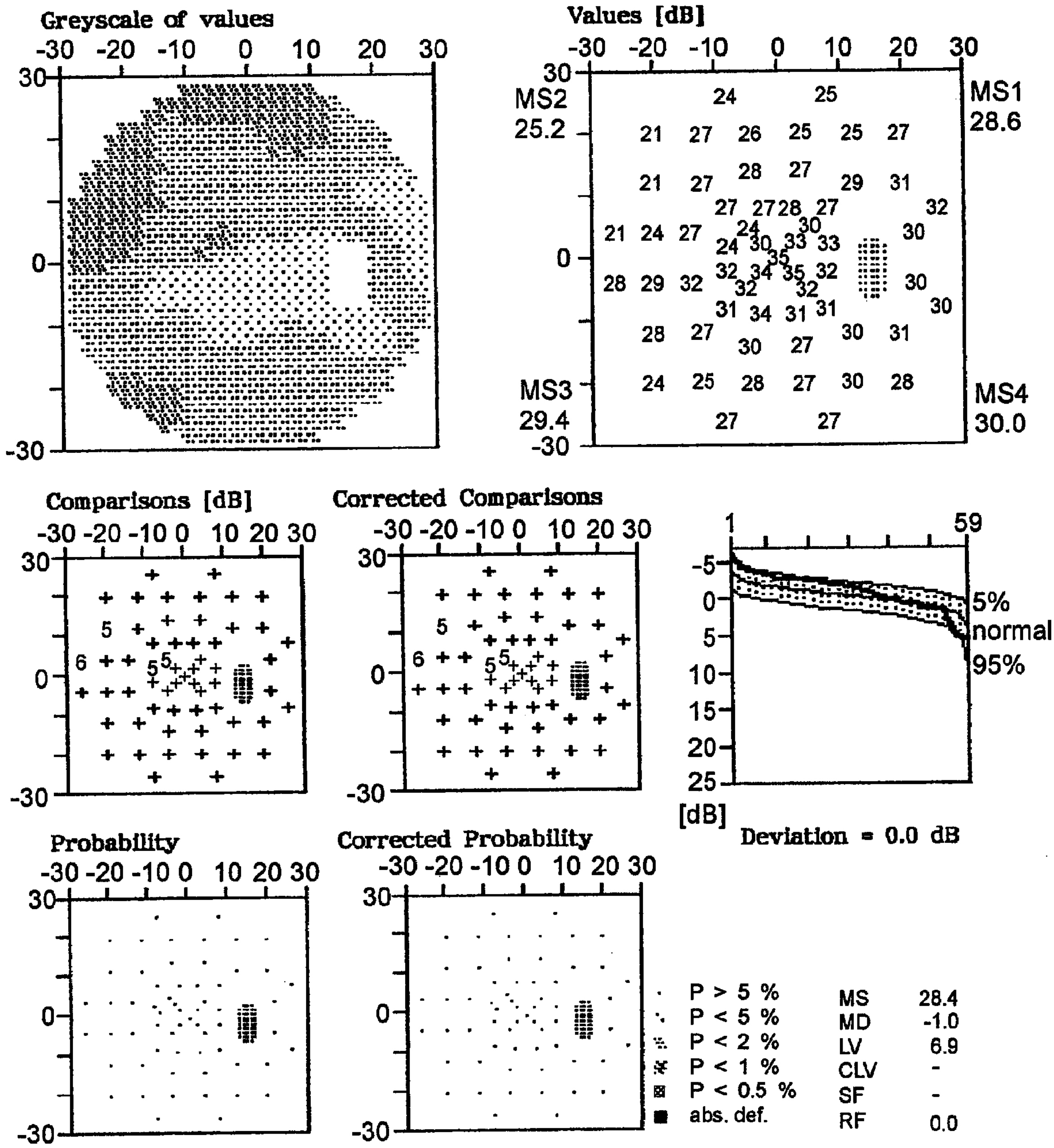
Greyscale of values
Values [dB]
MS1 28.6
MS2 25.2
MS3 29.4
MS4 30.0
Comparisons [dB]
Corrected Comparisons
5%
normal
95%
[dB]
Deviation = 0.0 dB
Probability
Corrected Probability
P > 5 %
P < 5 %
P < 2 %
P < 1 %
P < 0.5 %
abs. def.
MS 28.4
MD -1.0
LV 6.9
CLV -
SF -
RF 0.0

# Fig. 24

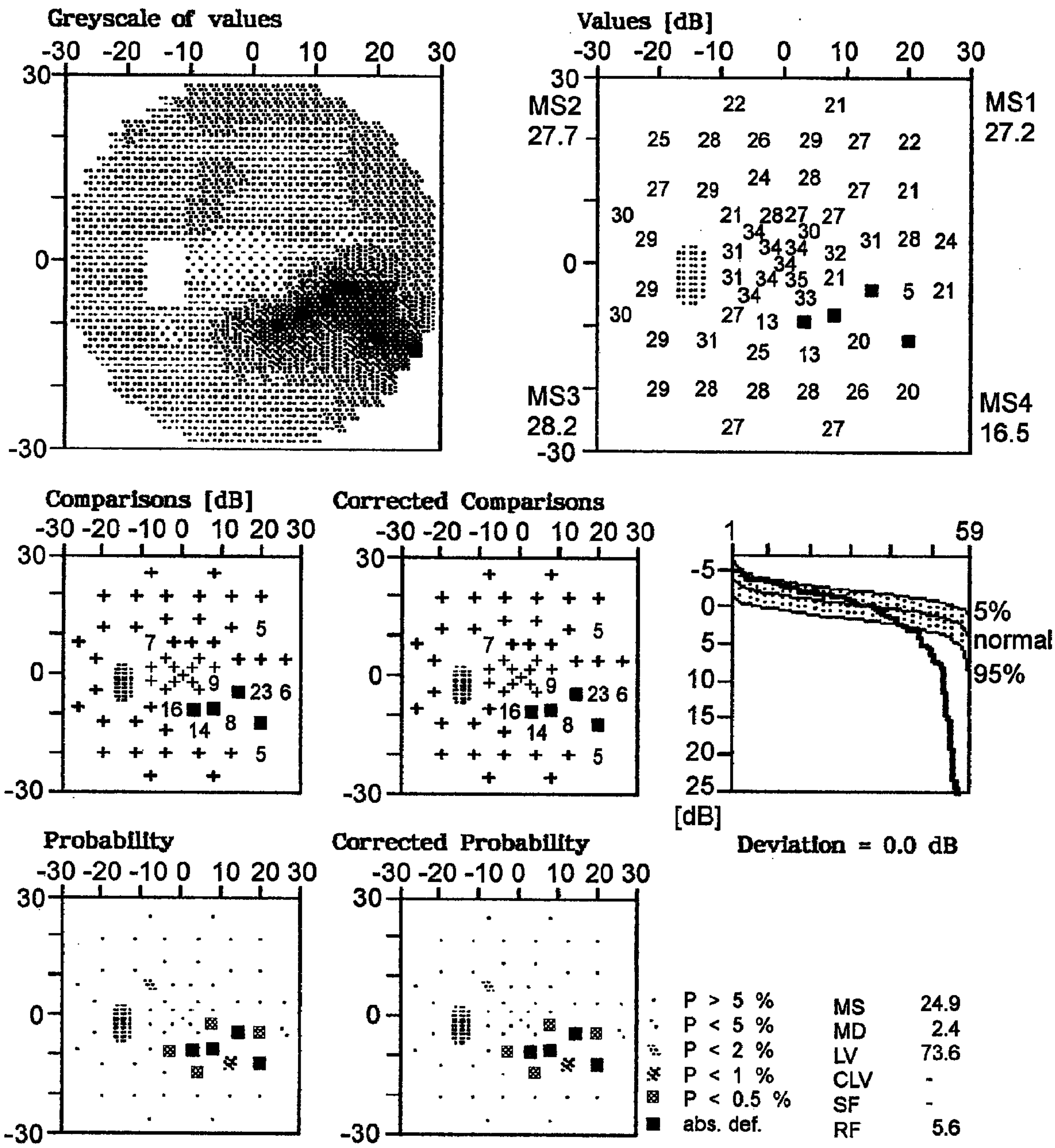
Greyscale of values
Values [dB]
MS1
27.2
MS2
27.7
MS3
28.2
MS4
16.5
Comparisons [dB]
Corrected Comparisons
5%
normal
95%
[dB]
Deviation = 0.0 dB
Probability
Corrected Probability
P > 5 %
P < 5 %
P < 2 %
P < 1 %
P < 0.5 %
abs. def.
MS 24.9
MD 2.4
LV 73.6
CLV -
SF -
RF 5.6

# Fig. 25

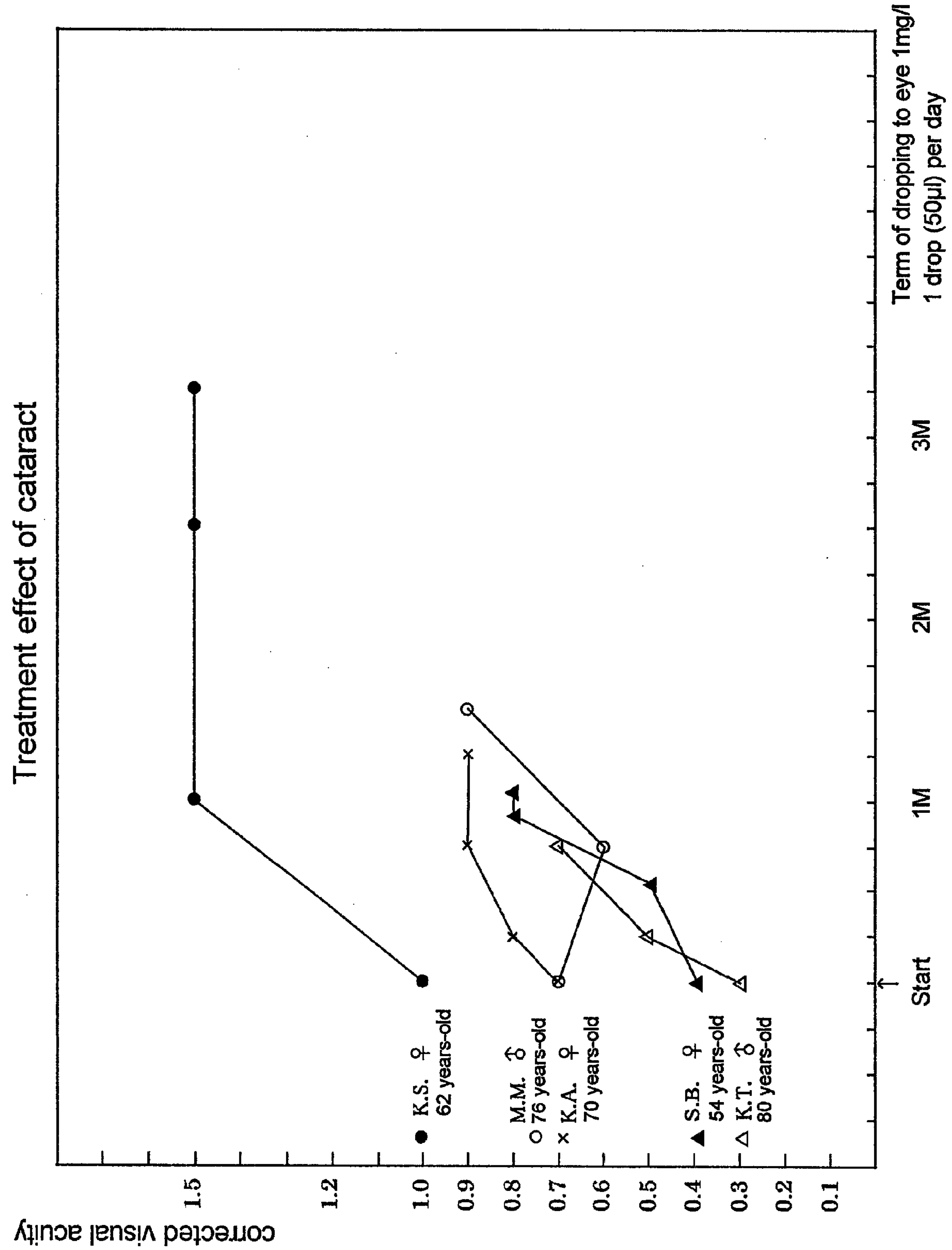
Treatment effect of cataract
corrected visual acuity
1.5
1.2
1.0
0.9
0.8
0.7
0.6
0.5
0.4
0.3
0.2
0.1
● K.S. ♀
62 years-old
○ M.M. ♂
76 years-old
× K.A. ♀
70 years-old
▲ S.B. ♀
54 years-old
△ K.T. ♂
80 years-old
Start
1M
2M
3M
Term of dropping to eye 1mg/l
1 drop (50μl) per day

# Fig. 26

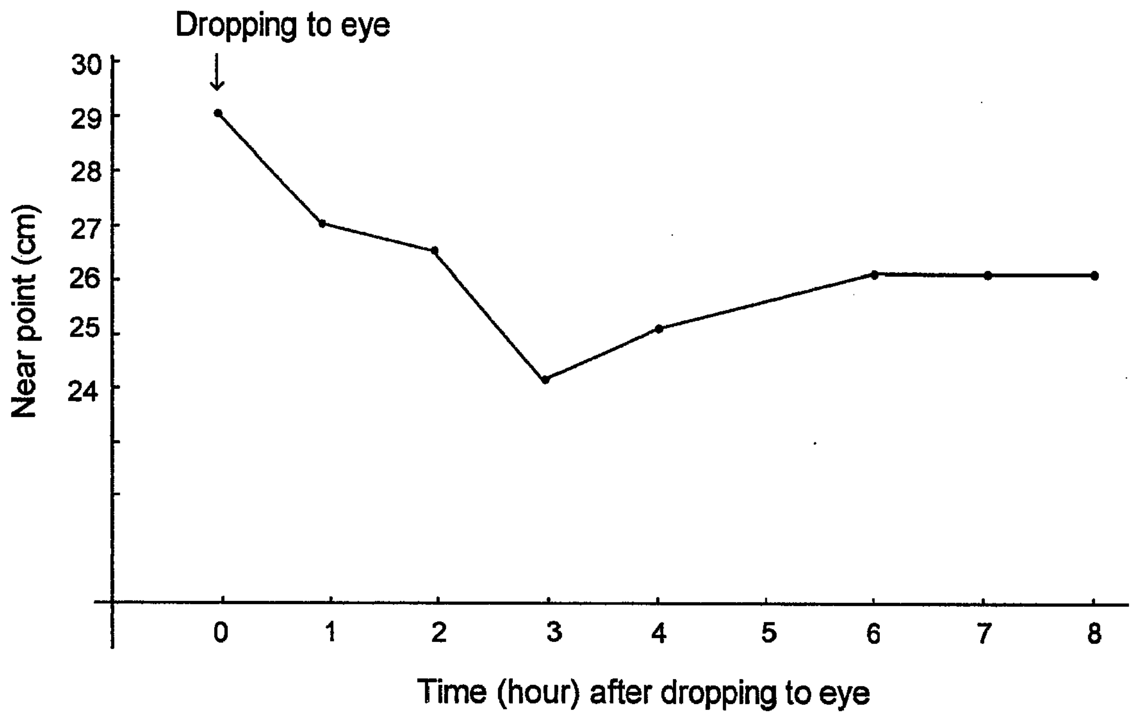
Dropping to eye
Near point (cm)
30
29
28
27
26
25
24
0
1
2
3
4
5
6
7
8
Time (hour) after dropping to eye

# Fig. 27

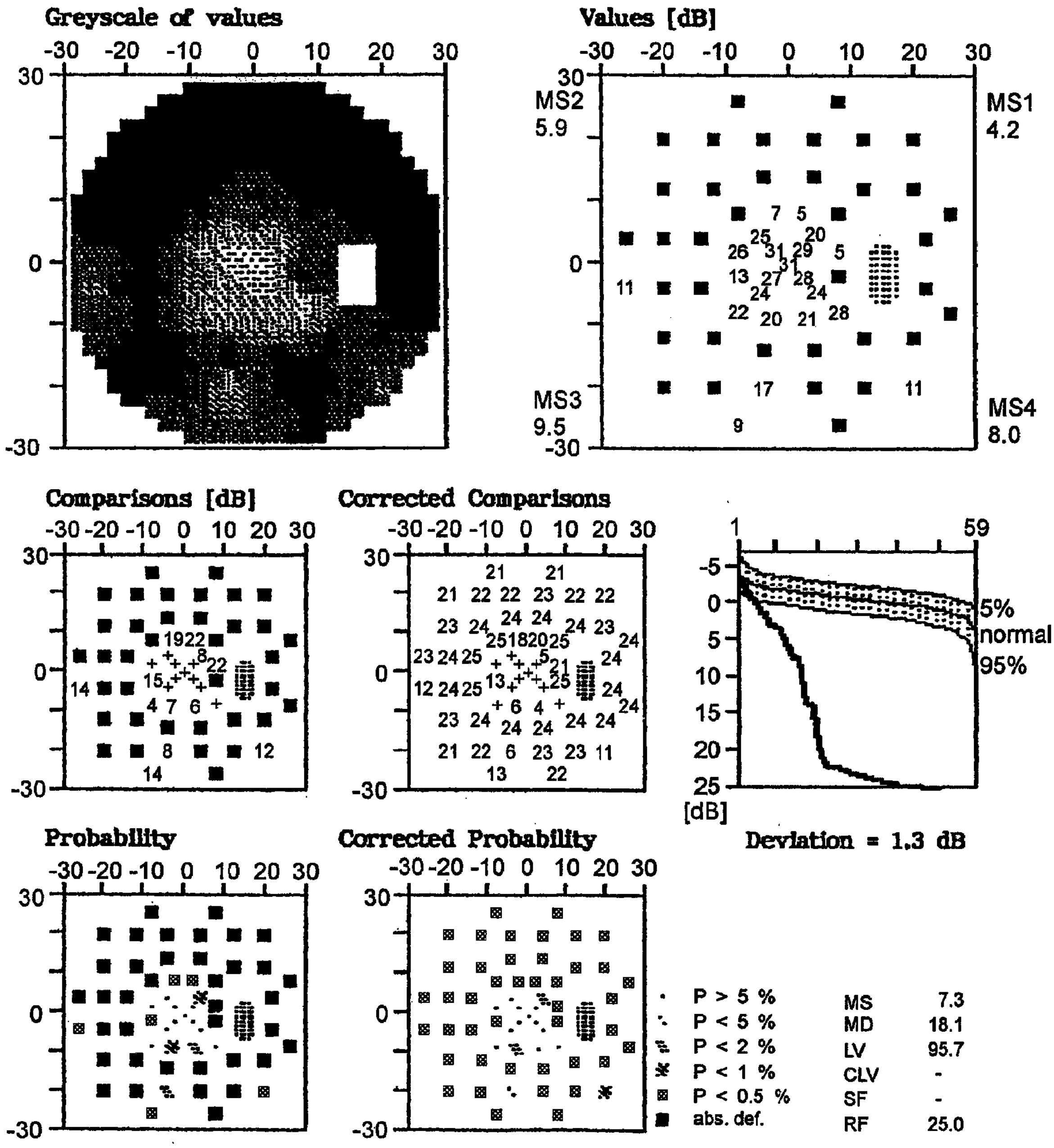
Greyscale of values
Values [dB]
MS2
5.9
MS1
4.2
MS3
9.5
MS4
8.0
Comparisons [dB]
Corrected Comparisons
5%
normal
95%
[dB]
Deviation = 1.3 dB
Probability
Corrected Probability
P > 5 %
P < 5 %
P < 2 %
P < 1 %
P < 0.5 %
abs. def.
MS 7.3
MD 18.1
LV 95.7
CLV -
SF -
RF 25.0

Fig. 28

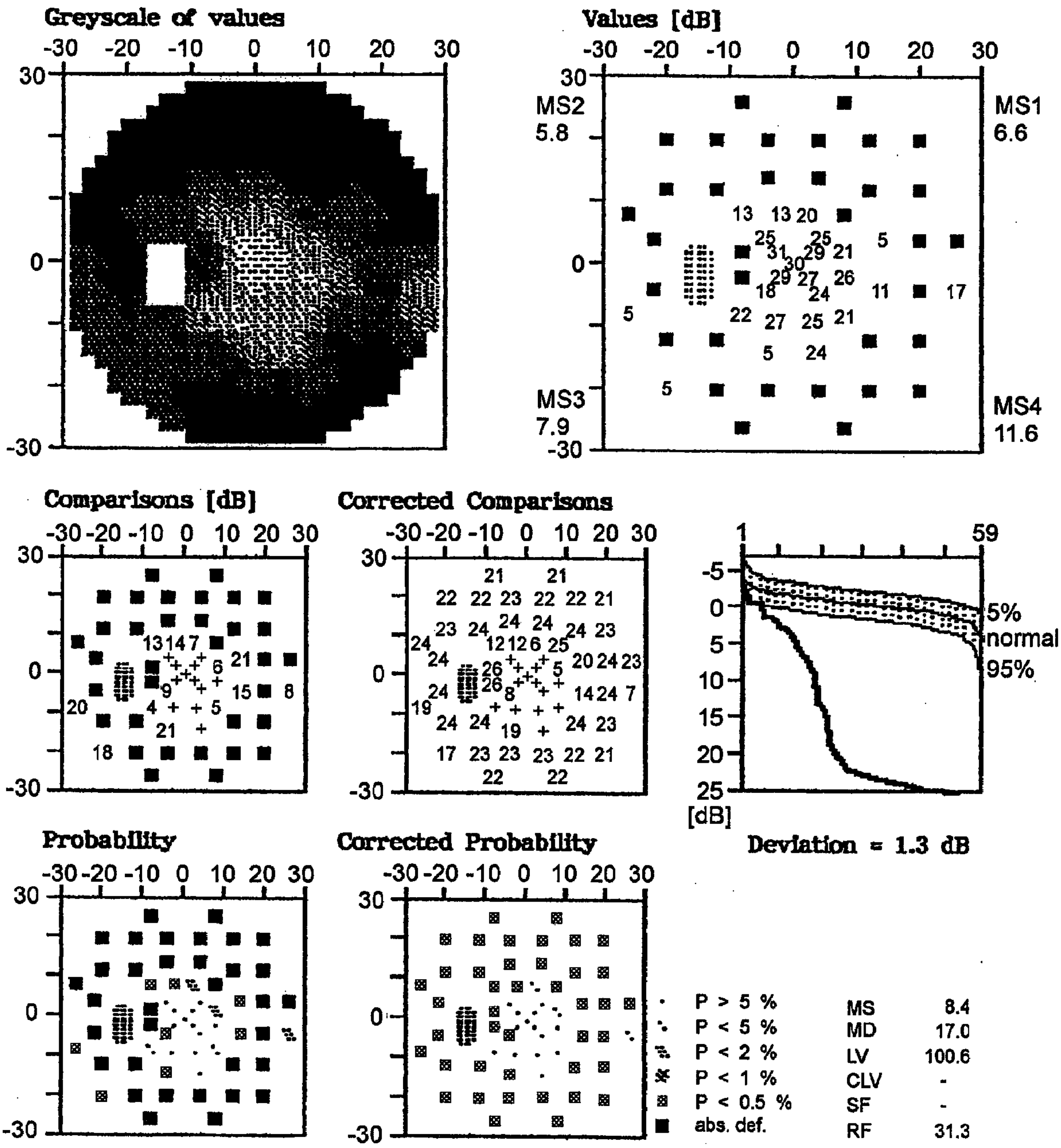
Greyscale of values
Values [dB]
MS2
5.8
MS1
6.6
MS3
7.9
MS4
11.6
Comparisons [dB]
Corrected Comparisons
5%
normal
95%
[dB]
Deviation = 1.3 dB
Probability
Corrected Probability
P > 5 %
P < 5 %
P < 2 %
P < 1 %
P < 0.5 %
abs. def.
MS 8.4
MD 17.0
LV 100.6
CLV -
SF -
RF 31.3

# Fig. 29

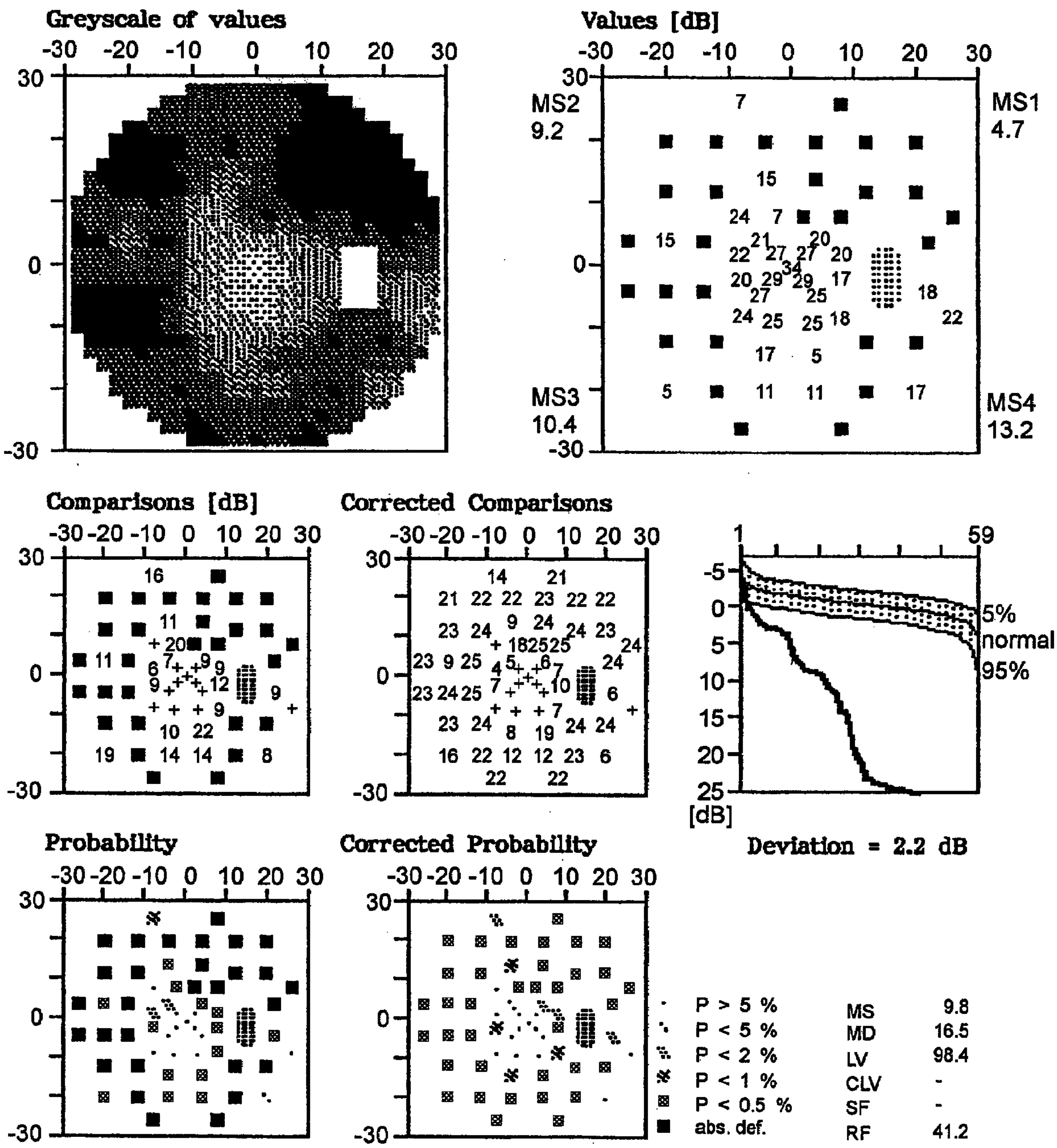
Greyscale of values
Values [dB]
MS1
4.7
MS2
9.2
MS3
10.4
MS4
13.2
Comparisons [dB]
Corrected Comparisons
5%
normal
95%
[dB]
Deviation = 2.2 dB
Probability
Corrected Probability
P > 5 %
P < 5 %
P < 2 %
P < 1 %
P < 0.5 %
abs. def.
MS 9.8
MD 16.5
LV 98.4
CLV -
SF -
RF 41.2

# Fig. 30

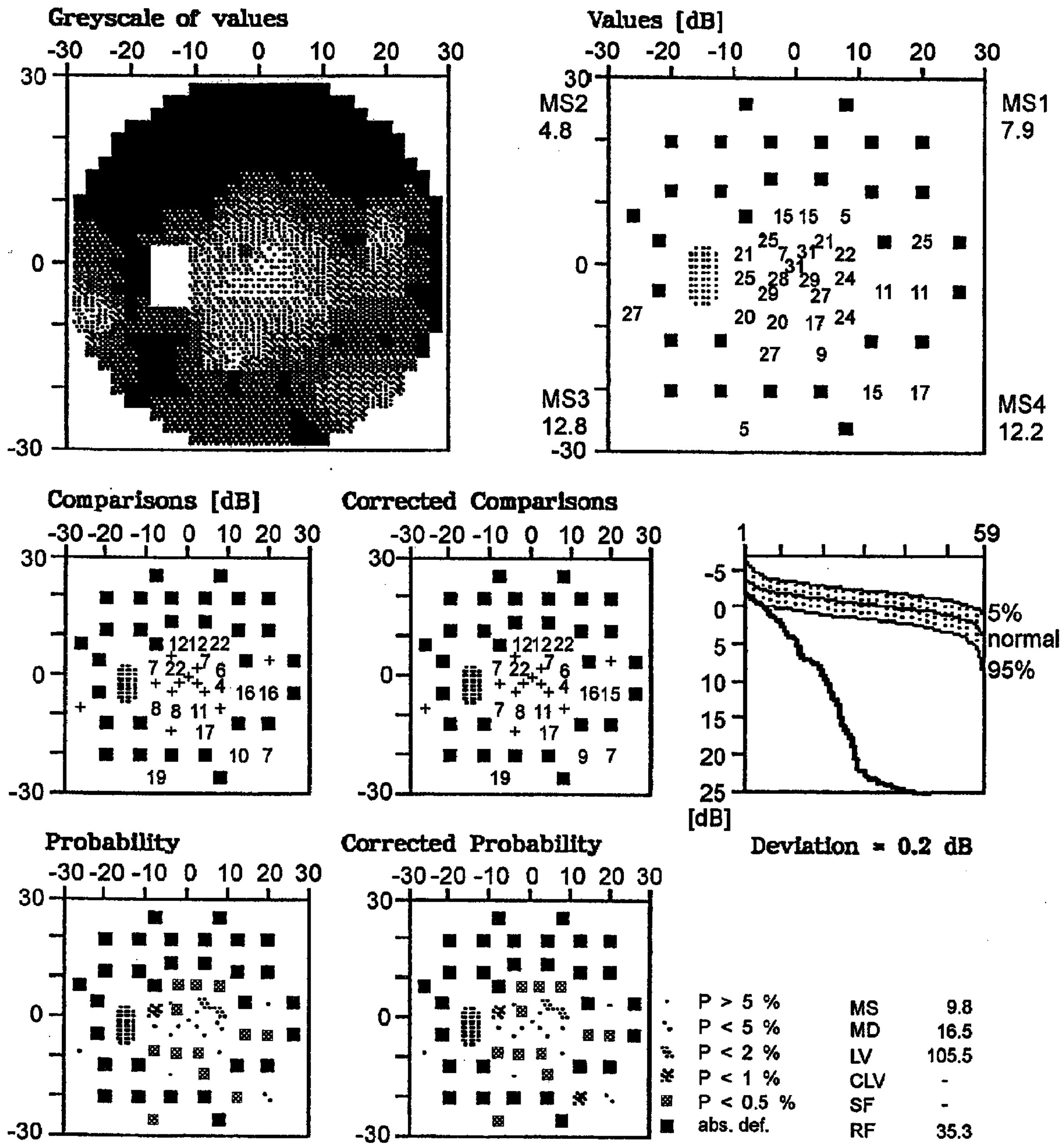
Greyscale of values
Values [dB]
MS1
7.9
MS2
4.8
MS3
12.8
MS4
12.2
Comparisons [dB]
Corrected Comparisons
5%
normal
95%
[dB]
Deviation = 0.2 dB
Probability
Corrected Probability
P > 5 %
P < 5 %
P < 2 %
P < 1 %
P < 0.5 %
abs. def.
MS 9.8
MD 16.5
LV 105.5
CLV -
SF -
RF 35.3

# Fig. 31

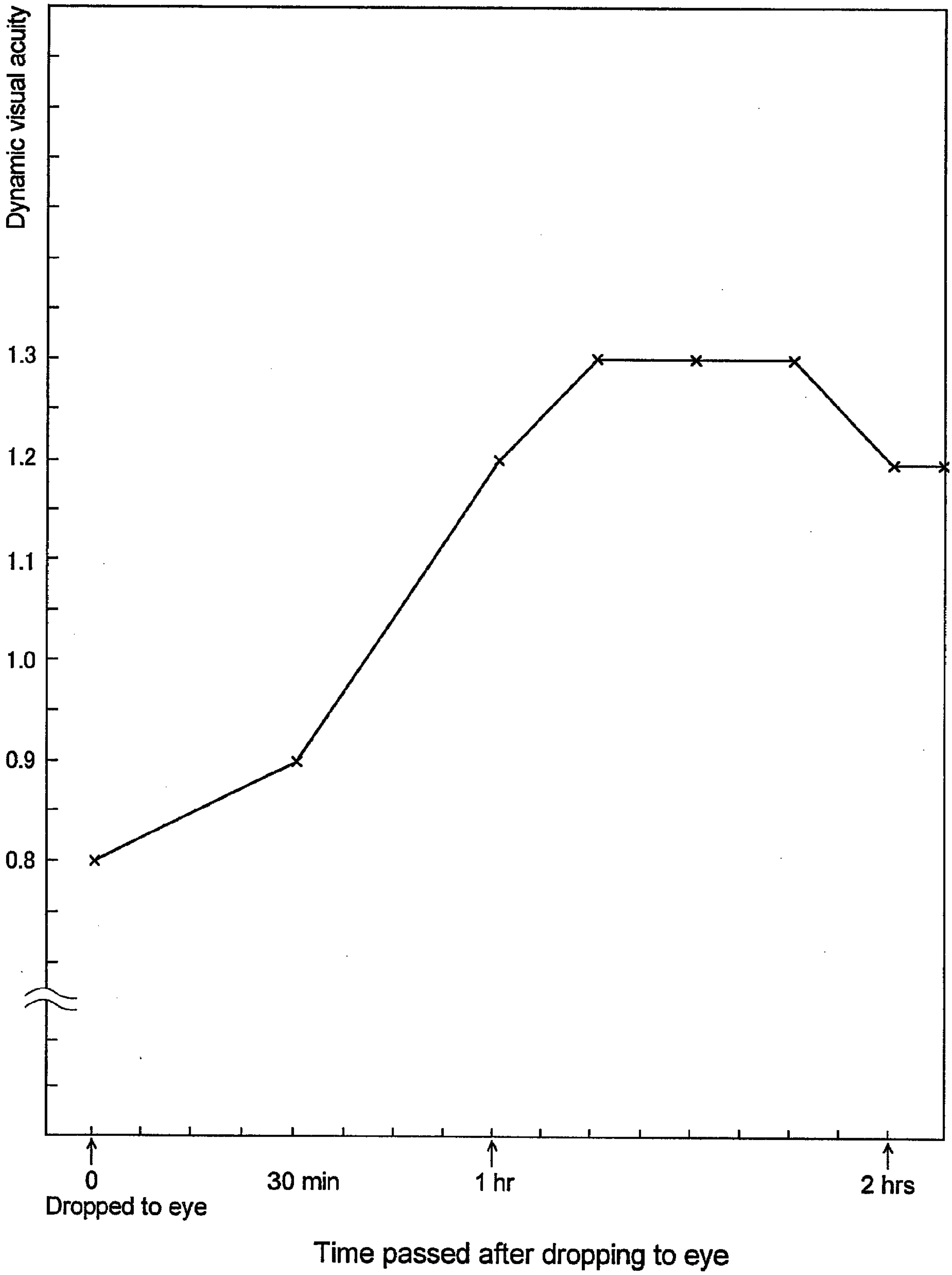
Dynamic visual acuity
1.3
1.2
1.1
1.0
0.9
0.8
0
Dropped to eye
30 min
1 hr
2 hrs
Time passed after dropping to eye

Europäisches Patentamt
European Patent Office
Office européen des brevets

# EUROPEAN SEARCH REPORT

Application Number
EP 10 17 8007

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 98/26784 A1 (UNIV SYDNEY [AU]; CHAMBERLAIN CORAL GWENDA [AU]; MCAVOY JOHNSTON WILLI) 25 June 1998 (1998-06-25)<br>* page 11, lines 4-8; claims 1,4-7 * | 1-3,9,10 | INV.<br>A61K31/56<br>A61K31/565<br>A61K31/568<br>A61K45/00<br>A61K9/06<br>A61K9/08<br>A61K47/40<br>A61P5/00<br>A61P27/02<br>A61P27/06<br>A61P27/10<br>C07J1/00 |
| X | WO 02/40032 A2 (UNIV WASHINGTON [US]) 23 May 2002 (2002-05-23)<br>* page 22, lines 23,34 * | 1-3 | |
| X | PESCH L A ET AL: "PROGESTERONE EFFECTS ON GALACTOSE METABOLISM IN PREPUBERTAL PATIENTS WITH CONGENITAL GALACTOSEMIA AND IN RATS MAINTAINED ON HIGH GALACTOSE DIETS",<br>JOURNAL OF CLINICAL INVESTIGATION, AMERICAN SOCIETY FOR CLINICAL INVESTIGATION, US,<br>vol. 39, no. 1,<br>1 January 1960 (1960-01-01), pages 178-184, XP009144483,<br>ISSN: 0021-9738<br>* page 181, column 1, lines 17-20 *<br>* page 183, column 1, lines 4-9 * | 1,2 | TECHNICAL FIELDS SEARCHED (IPC)<br>A61K |
| X | EP 1 177 787 A2 (PFIZER PROD INC [US]) 6 February 2002 (2002-02-06)<br>* paragraphs [0013], [0014], [0028]; claims 8,10; example 3 * | 1,2 | |
| Y | US 4 877 774 A (PITHA ET AL) 31 October 1989 (1989-10-31)<br>* column 3, line 8 - line 10 *<br>* column 3, line 35 - line 37 *<br>* column 5, line 28 - line 33; claims 1-10 * | 5-8 | |
| | -/-- | | |

The present search report has been drawn up for all claims

| Place of search | Date of completion of the search | Examiner |
|---|---|---|
| Munich | 18 February 2011 | Allnutt, Sarah |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 17 8007

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 98/03180 A2 (UNIV MANCHESTER [GB]; FERGUSON MARK WILLIAM JAMES [GB]; ASHCROFT GILLI) 29 January 1998 (1998-01-29)<br>* page 14, paragraph 2 *<br>* page 15, paragraph 3 *<br>* page 10, paragraph 2 *<br>----- | 5-8 | |

TECHNICAL FIELDS SEARCHED (IPC)

The present search report has been drawn up for all claims

3

| Place of search | Date of completion of the search | Examiner |
|---|---|---|
| Munich | 18 February 2011 | Allnutt, Sarah |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 17 8007

This annex lists the patent family members relating to the patent documents cited in the above-mentioned European search report.
The members are as contained in the European Patent Office EDP file on
The European Patent Office is in no way liable for these particulars which are merely given for the purpose of information.

18-02-2011

| Patent document cited in search report | | Publication date | Patent family member(s) | | | Publication date |
|---|---|---|---|---|---|---|
| WO 9826784 | A1 | 25-06-1998 | AT | 309842 | T | 15-12-2005 |
| | | | DE | 69734677 | D1 | 22-12-2005 |
| | | | DE | 69734677 | T2 | 10-08-2006 |
| | | | EP | 1003522 | A1 | 31-05-2000 |
| | | | JP | 2001506638 | T | 22-05-2001 |
| | | | US | 6258856 | B1 | 10-07-2001 |
| WO 0240032 | A2 | 23-05-2002 | AU | 2889102 | A | 27-05-2002 |
| EP 1177787 | A2 | 06-02-2002 | AU | 5767101 | A | 31-01-2002 |
| | | | CA | 2354208 | A1 | 28-01-2002 |
| | | | HU | 0103078 | A2 | 29-05-2002 |
| | | | JP | 2002087992 | A | 27-03-2002 |
| | | | KR | 20020010504 | A | 04-02-2002 |
| | | | ZA | 200106160 | A | 27-01-2003 |
| US 4877774 | A | 31-10-1989 | NONE | | | |
| WO 9803180 | A2 | 29-01-1998 | AT | 279916 | T | 15-11-2004 |
| | | | AU | 734465 | B2 | 14-06-2001 |
| | | | AU | 3628897 | A | 10-02-1998 |
| | | | CA | 2261263 | A1 | 29-01-1998 |
| | | | CA | 2632790 | A1 | 29-01-1998 |
| | | | DE | 69731302 | D1 | 25-11-2004 |
| | | | DE | 69731302 | T2 | 16-02-2006 |
| | | | EP | 0930876 | A2 | 28-07-1999 |
| | | | ES | 2229376 | T3 | 16-04-2005 |
| | | | HK | 1021319 | A1 | 25-02-2005 |
| | | | JP | 2000515523 | T | 21-11-2000 |
| | | | JP | 2008174566 | A | 31-07-2008 |
| | | | JP | 2009051844 | A | 12-03-2009 |
| | | | PT | 930876 | E | 31-01-2005 |
| | | | US | 2002042401 | A1 | 11-04-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5521168 A
- WO 0240032 A2


### Non-patent literature cited in the description


- *Neurobiology of Disease,* 2002, vol. 9, 282-293